(19) **Europäisches Patentamt / European Patent Office / Office européen des brevets**

(11) **EP 4 740 940 A1**

(12) # EUROPEAN PATENT APPLICATION
published in accordance with Art. 153(4) EPC

(43) Date of publication:
**13.05.2026 Bulletin 2026/20**

(21) Application number: **24838946.2**

(22) Date of filing: **11.07.2024**

(51) International Patent Classification (IPC):
*A61K 9/50* (2006.01)  *A61K 9/72* (2006.01)
*A61K 47/46* (2006.01)  *A61K 47/18* (2017.01)
*A61K 47/26* (2006.01)  *A61K 31/438* (2006.01)
*A61P 31/06* (2006.01)

(86) International application number:
**PCT/ES2024/070439**

(87) International publication number:
**WO 2025/012506 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.07.2023 ES 202330580**

(71) Applicants:
• **Universidade de Santiago de Compostela**
  **15782 Santiago de Compostela, A Coruña (ES)**
• **University of Szeged**
  **6720 Szeged (HU)**

(72) Inventors:
• **CSABA, Noemi**
  **15782 Santiago de Compostela (A Coruña) (ES)**
• **ROBLA ÁLVAREZ, Sandra**
  **15782 Santiago de Compostela (A Coruña) (ES)**
• **VALVERDE FRAGA, Lorena**
  **15782 Santiago de Compostela (A Coruña) (ES)**
• **SANCHEZ POZA, Sandra**
  **15782 Santiago de Compostela (A Coruña) (ES)**
• **AMBRUS, Rita**
  **6726 Szeged (HU)**
• **CSÓKA, Ildikó**
  **6772 Deszk (HU)**

(74) Representative: **ABG Intellectual Property Law, S.L.**
  **Avenida de Burgos, 16D**
  **Edificio Euromor**
  **28036 Madrid (ES)**

(54) **POLLEN PARTICLES AS CARRIERS FOR PULMONARY DELIVERY**

(57)    The present invention relates to microparticles for pulmonary release that comprise a purified pollen capsule, which comprises an intine layer and an exine layer, and at least one nanosystem encapsulated in said capsule. Said nanosystem comprises a pharmaceutically active compound, wherein the exine layer is coated with a pharmaceutically acceptable excipient. The present invention further relates to a method of obtaining said microparticles and medical uses thereof, in particular for the treatment of tuberculosis.

**Description**

**Field of the Invention**

The present invention is comprised within the field of purified pollen particles as carriers for pulmonary release.

**Background of the Invention**

Standard treatment for tuberculosis (TB) caused by *Mycobacterium tuberculosis* includes therapy with oral antibiotics. The low solubility and high metabolism of drugs in the oral treatment of tuberculosis leads to the use of prolonged therapies that favor the appearance of resistances, hindering patient adherence. Furthermore, the appearance of mycobacterial strains which are resistant to medicinal products requires more medicinal products and longer treatments. Alternatively, pulmonary drug delivery offers direct access to lung epithelium and alveolar macrophages, preventing side effects associated with oral delivery. However, several factors, such as physical barriers, pulmonary defense mechanisms such as macrophages, alveolar enzymes, and mucociliary clearance, constitute an obstacle in pulmonary drug release, so there are currently no inhalable antitubercular formulations available for clinical use.

Rifabutin, a class II drug according to the Biopharmaceutical Classification System (BCS), exhibits poor dissolution and limited absorption. This compound shares structural and activity similarities with rifampicin, but has greater antimycobacterial activity and extensive tissue distribution. However, its large volume of distribution and low oral bioavailability (less than 20% reaches systemic circulation) result in low plasma concentrations. Encapsulation of such drugs within nanoparticles could be an effective solution, as nanoparticles with sizes of about 200 nm are considered suitable for pulmonary delivery. However, nanoparticles exhibit low inertia and are exhaled before reaching the airways. This challenge could be overcome by incorporating nanoparticles into micrometer-sized particles, serving as a delivery system to make it easier for them to reach alveolar macrophages.

Strategies using microparticle delivery in the form of dry powder inhalers (DPIs) have shown to be effective in the generation of inhalable particles for the treatment of TB [Mehta, P.; Bothiraja, C.; Kadam, S.; Pawar, A. Potential of Dry Powder Inhalers for Tuberculosis Therapy: Facts, Fidelity and Future. Artif. Cells, Nanomedicine, Biotechnol. 2018, 46, S791-S806, doi:10.1080/21691401.2018.1513938].

The publication by Robla, S., et al. ("A ready-to-use dry powder formulation based on protamine nanocarriers for pulmonary drug release", European Journal of Pharmaceutical Sciences, 2023, 185, 106442) describes rifabutin-loaded protamine nanocapsules for pulmonary release. The nanocapsules are incorporated into microparticles by lyophilization with mannitol, obtaining an inhalational dry powder formulation for the treatment of tuberculosis.

These approaches use particle engineering techniques such as spray drying and lyophilization to produce suitable inhalable particles.

In this context, pollen grains have emerged in recent years as viable candidates for drug encapsulation and delivery due to their micrometer-sized structure.

Pollen grains exhibit a uniform size and essentially comprise genetic material contained in a cytoplasm (sporoplasm), which is coated by a first inner layer called intine and a second layer called exine.

The exine of pollen particles has on its surface an additional lipid layer, a complex mixture of proteins, lipids, and other molecules (known as "pollenkitt"). The intine generally consists of cellulose, while the exine is composed of a proteinaceous material called sporopollenin the exact composition of which is not known. The exine is an extremely strong layer that is stable in acidic and basic conditions and has high porosity. Given these properties, various technologies that allow isolating exine, i.e., hollowing out the interior of exine, removing its intine and genetic material, as well as cleaning the outer surface of the lipid layer or pollenkitt, have been tested.

These natural structures can be easily processed to create a low-density platform with a large inner cavity, suitable for drug delivery by means of encapsulation of both polar and non-polar drugs, providing them with protection. Furthermore, it has been shown that the use of sporopollenin improves drug adhesion capacity and increases contact surface between drug and mucosa [Diego-Taboada, A.; Beckett, S.T.; Atkin, S.L.; Mackenzie, G. Hollow Pollen Shells to Enhance Drug Release. Pharmaceutics 2014, 6, 80-96, doi:10.3390/pharmaceutics6010080].

To date, the potential use of pollen particles for pulmonary drug delivery remains unexplored. While pollen is deposited primarily in the oropharynx, prolonged exposure, particularly to specific proteins, can cause sensitization and the development of allergies and asthma, both in the lower respiratory tract and following ingestion in the form of food allergies. Allergens can be removed by means of chemical treatment methods, resulting in purified, allergen-free pollen particles which are suitable for drug delivery.

Document WO2018146365 is a patent application focusing on purified pollen particles and use thereof in encapsulated active ingredient delivery. It describes a purified pollen particle which comprises an intine layer, an exine layer, and a nanosystem, with the removal of the lipid layer (pollenkitt). This document does not describe coating the surface of the pollen particle with a pharmaceutically acceptable excipient or a specific system for pulmonary delivery.

Document WO2021183735 is a patent application focusing on protein-free pollen capsules for delivering active compounds to the lungs. It mentions organic molecules having a size of 50-2500 Da, including antimicrobials, and the use of transfection agents such as polymers, lipids, and polypeptide vesicles. Carriers such as liposomes or carrier proteins can be combined with the active compounds. However, the document lacks details concerning the composition of the pollen particles and does not specify a pharmaceutically acceptable excipient that covers the surface of the pollen particles.

Document WO2005000280 is a patent application focusing on an active substance bound to a support with an exine coating of spores, potentially sporopollenin. It describes a purified pollen particle that lacks an intine layer. It describes a formulation for pulmonary delivery in which the active compound is encapsulated within the exine shell of the pollen particle. However, document WO2005000280 does not mention the presence of an intine layer or the use of nanosystems for encapsulating the active compound.

Application WO2018146365A1 describes the incorporation of nanosystems in hollow spores but does not mention the use thereof in pulmonary delivery.

Document US5275819A discloses insulin- or angiotensin-loaded pollen grain microparticles, in which the pollen grains have an outer coating to provide a pulsed release of the active substance.

Therefore, there are currently no formulations for pulmonary delivery, particularly inhalable antitubercular formulations, available for clinical use.

**Brief Description of the Invention**

The authors of the present invention have developed low-density, micrometer-sized particles for depositing drugs deep in the lungs. The microparticles are based on pollen particles which provide protection to an encapsulated drug and exhibit an aerodynamic distribution suitable for use as a system for releasing drugs in the lung.

The microparticles of the present invention are a drug release system that may be considered a dual-carrier release system. The first carrier allows the drug to be transported to the airways and, once at its destination, the second carrier allows cell penetration. The first carrier is purified pollen particles, i.e., pollen without the outer lipid layer (pollenkitt) and without sporoplasm, which comprise an intine and exine layer, wherein the exine is preferably functionalized with a pulmonary excipient so that the particles are suitably distributed in the pulmonary airways (excipient which allows reducing their aerodynamic diameter and increasing the fine particle fraction, allowing the particles to reach the alveoli and achieve improved pulmonary distribution). The purified pollen particles, referred to in the present disclosure as capsules, transport what could be considered a second carrier (a nanosystem), comprising the drug. This nanosystem allows the drug to go through the cell membrane.

Therefore, a first aspect of the present invention relates to a microparticle for pulmonary release comprising:

- a purified pollen capsule, which comprises an intine layer and an exine layer; and

- at least one nanosystem encapsulated in said capsule, wherein the nanosystem comprises a pharmaceutically active compound;

wherein the exine layer is coated with a pharmaceutically acceptable excipient.

A second aspect of the present invention relates to a method for the preparation of the microparticle of the first aspect, characterized in that it comprises:

(a) a washing stage which comprises washing a pollen particle;

(b) a stage which comprises treating the pollen particle obtained in the preceding stage with an acidic medium to obtain hollow purified pollen capsules;

(c) a stage which comprises incubating the hollow capsules obtained in the preceding stage with a nanosystem comprising a pharmaceutically active compound; and

(d) a stage which comprises coating the resulting capsules with a pharmaceutically acceptable excipient.

A third aspect of the present invention relates to a microparticle for pulmonary release, characterized in that it is obtained according to the method of the second aspect.

A fourth aspect of the present invention relates to a pharmaceutical composition, characterized in that it comprises a microparticle according to the first or third aspect, and a pharmaceutically acceptable excipient.

A fifth aspect of the present invention relates to a microparticle according to the first or third aspect, or a pharmaceutical composition according to the fourth aspect, for use as a medicinal product.

A sixth aspect of the present invention relates to a microparticle according to the first or third aspect, or a pharmaceutical composition according to the fourth aspect, for use in the treatment of lung-related diseases.

**Brief Description of the Figures**

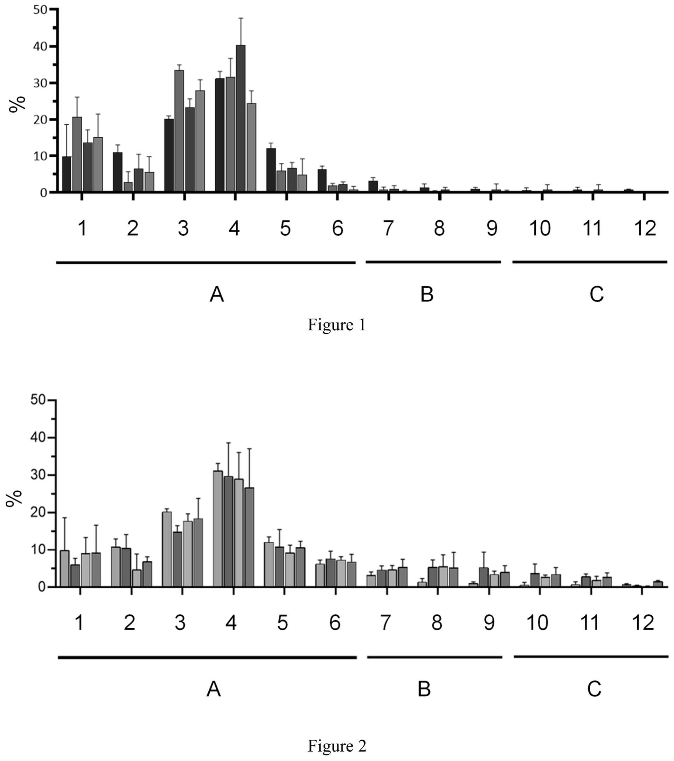

Figure 1. *In vitro* aerodynamic deposition of hollow and defatted *H. annuus* and *M. chamomilla* pollen capsules in the Andersen Cascade Impactor. Average values ± standard deviation, n = 3. Y-axis, percentage. X-axis, A: extrathoracic region; B: tracheobronchial region; C: pulmonary region; 1: Inhaler; 2: Mouth; 3: Trachea; 4: stage -1; 5: stage 0; 6: stage 1; 7: stage 2; 8: stage 3; 9: stage 4; 10: stage 5; 11: stage 6; 12: filter. Each group contains 4 columns, the leftmost column represents hollow *M. chamomilla* capsules, the second column represents defatted *M. chamomilla* capsules, the third column represents hollow *H. annuus* capsules, and the rightmost column represents defatted *H. annuus* capsules.

Figure 2. *In vitro* aerodynamic deposition in the Andersen Cascade Impactor of mannitol- and/or leucine-coated *M. chamomilla* capsules. Average values ± standard deviation, n = 3. Y-axis, percentage. X-axis, A: extrathoracic region; B: tracheobronchial region; C: pulmonary region; 1: Inhaler; 2: Mouth; 3: Trachea; 4: stage -1; 5: stage 0; 6: stage 1; 7: stage 2; 8: stage 3; 9: stage 4; 10: stage 5; 11: stage 6; 12: filter. Each group contains 4 columns, the leftmost column represents uncoated *M. chamomilla* capsules, the second column represents mannitol-coated *M. chamomilla* capsules, the third column represents leucine-coated *M. chamomilla* capsules, and the rightmost column represents mannitol- and leucine-coated *M. chamomilla* capsules.

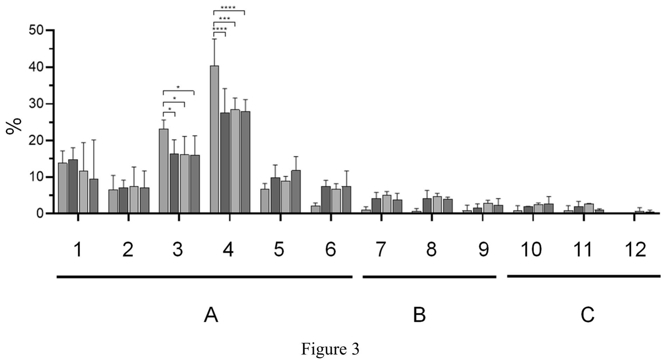

Figure 3. *In vitro* aerodynamic deposition in the Andersen Cascade Impactor of mannitol- and/or leucine-coated *H. annuus* capsules. Average values ± standard deviation, n = 3. Y-axis, percentage. X-axis, A: extrathoracic region; B: tracheobronchial region; C: pulmonary region; 1: Inhaler; 2: Mouth; 3: Trachea; 4: stage -1; 5: stage 0; 6: stage 1; 7: stage 2; 8: stage 3; 9: stage 4; 10: stage 5; 11: stage 6; 12: filter. Each group contains 4 columns, the leftmost column represents uncoated *H. annuus* capsules, the second column represents leucine-coated *H. annuus* capsules, the third column represents mannitol-coated *H. annuus* capsules, and the rightmost column represents mannitol- and leucine-coated *H. annuus* capsules.

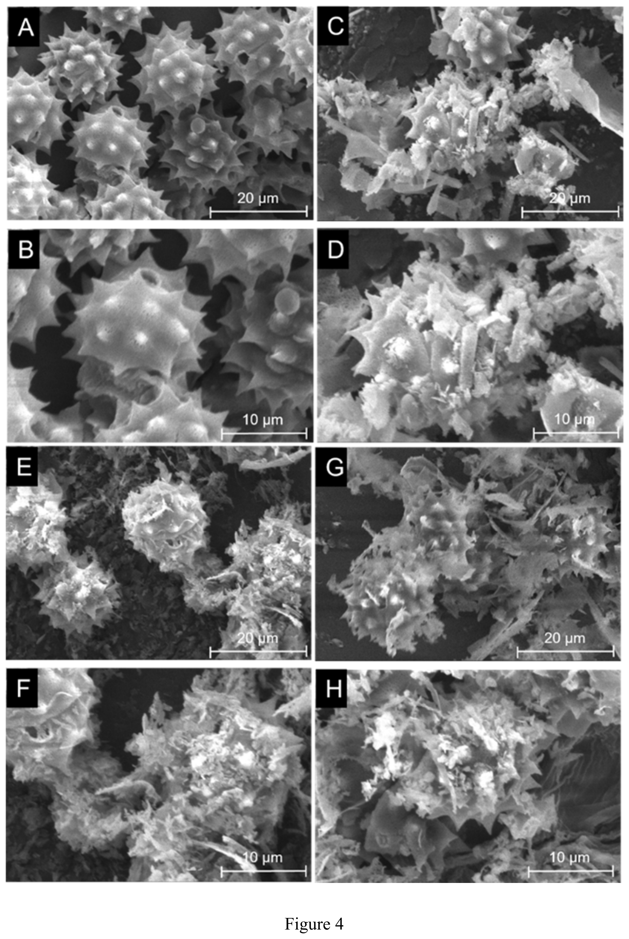

Figure 4. Scanning electron microscopy (SEM) images of hollow *M. chamomilla* capsules (A, B), after surface coating by lyophilization with mannitol (C, D), leucine (E, F), and combination thereof (G, H) at x2.0K (A, C, E, G) and x3.5K (B, D, F, H).

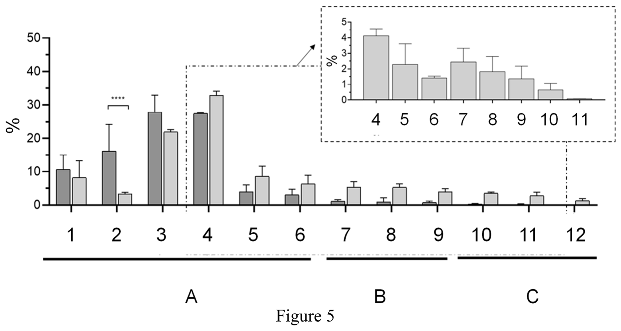

Figure 5. *In vitro* aerodynamic deposition of capsules according to the invention and uncoated capsules in the Andersen Cascade Impactor. Average values ± standard deviation, n = 3. Y-axis, percentage. Y-axis of the graph in the box, percentage of rifabutin. X-axis, A: extrathoracic region; B: tracheobronchial region; C: pulmonary region; 1: Inhaler; 2: Mouth; 3: Trachea; 4: stage -1; 5: stage 0; 6: stage 1; 7: stage 2; 8: stage 3; 9: stage 4; 10: stage 5; 11: stage 6; 12: filter. Each group contains 2 columns, the leftmost column represents uncoated *M. chamomilla* capsules with protamine nanosystems with 5% (w/w) of rifabutin, whereas the rightmost column represents *M. chamomilla* capsules with protamine nanosystems with 5% (w/w) of rifabutin, coated with a combination of mannitol and leucine.

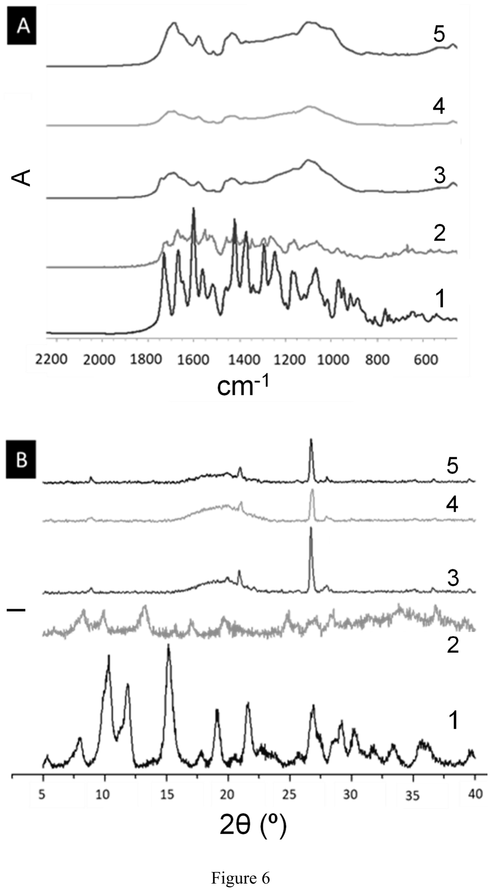

Figure 6. (A) FTIR and (B) X-ray diffraction spectra (XRPD) of: 1, rifabutin; 2, purified pollen particles with rifabutin; 3, purified pollen particles with protamine nanosystems comprising rifabutin; 4, purified pollen particles with nanosystems without rifabutin; and 5, purified *M. chamomilla* pollen particles. In Figure A: Y-axis, absorbance; X-axis, wavenumber (in cm$^{-1}$). In Figure B: Y-axis, relative intensity (%); X-axis, 2 theta (in degrees).

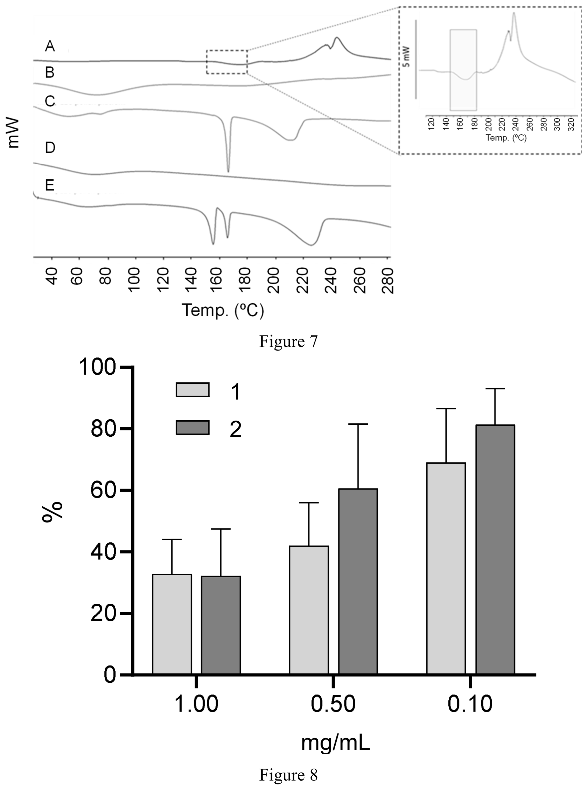

Figure 7. DSC thermogram of hollow and nanosystem-loaded *M. chamomilla* pollen particles without coating and with a mannitol (MAN)/leucine (LEU) surface coating. Y-axis, heat flux. Curve A: Rifabutin; Curve B, *M. chamomilla* pollen particles; Curve C, MAN/LEU-coated *M. chamomilla* particles; Curve D, *M. chamomilla* particles with protamine nanosystems comprising rifabutin; and Curve E, MAN/LEU-coated *M. chamomilla* microparticles with protamine nanosystems comprising rifabutin according to the invention.

Figure 8. MTT results of macrophage cell line Raw264.7 after treatment with the capsules the invention (loaded with 5% rifabutin) at different concentrations (100-1000 μg/mL) for 24 h. The results are shown as average values ± standard deviation, n = 3. Y-axis, viability expressed in percentage. X-axis, concentration expressed in mg/mL.

Legend, 1: *M. chamomilla* with a protamine nanosystem comprising 5% rifabutin (w/w); 2: protamine nanosystem comprising 5% rifabutin (w/w).

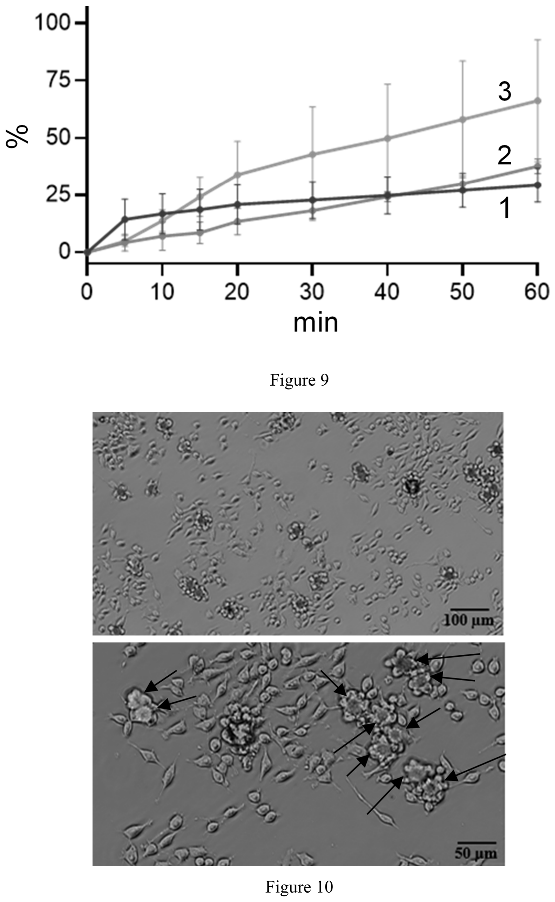

Figure 9. *In vitro* dissolution of: 1, rifabutin from the microparticles of the invention (5% rifabutin (w/w) in a protamine nanosystem encapsulated in leucine- and mannitol-coated *M. chamomilla* capsules; 2, rifabutin; and 3, rifabutin in non-encapsulated nanosystems. The data represents average ± standard deviation, n=3 independent measurements.

Figure 10. Fluorescence microscope images of lung epithelial cells A549 (top) and RAW 264.7 cells (bottom) cultured in µ-Slide I for 24 h after perfusion with purified pollen capsules.

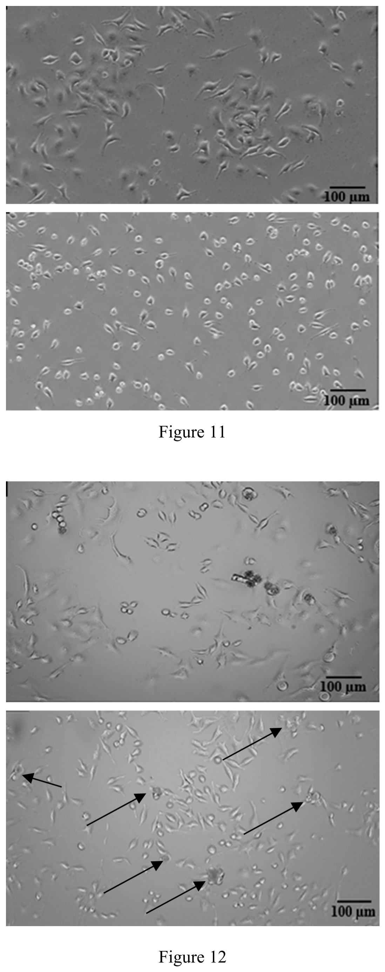

Figure 11. Fluorescence microscope images of lung epithelial cells A549 (top) and RAW 264.7 cells (bottom) cultured in µ-Slide I for 24 h after perfusion with DiD-labeled chitosan nanosystems.

Figure 12. Fluorescence microscope images of lung epithelial cells A549 (top) and RAW 264.7 cells (bottom) cultured in µ-Slide I for 24 h after perfusion with microparticles according to the invention.

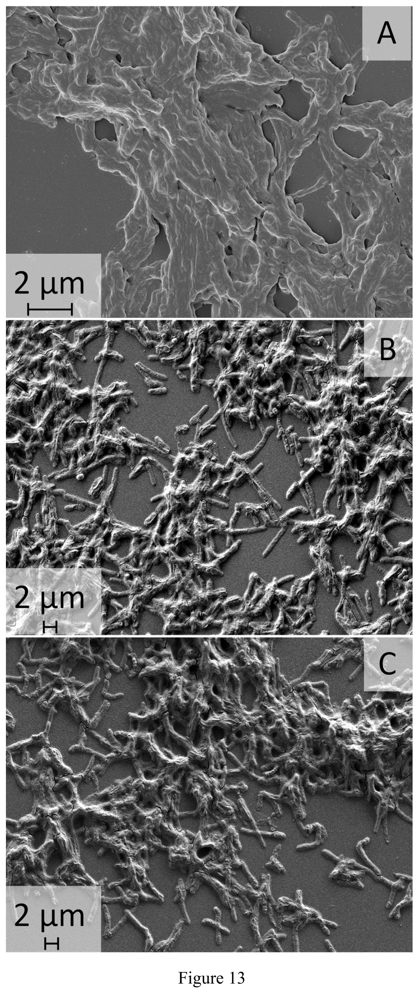

Figure 13. SEM images of *M. smegmatis* control biofilm (A), control after treatment with rifabutin (B), and control after treatment with nanosystems without rifabutin (C).

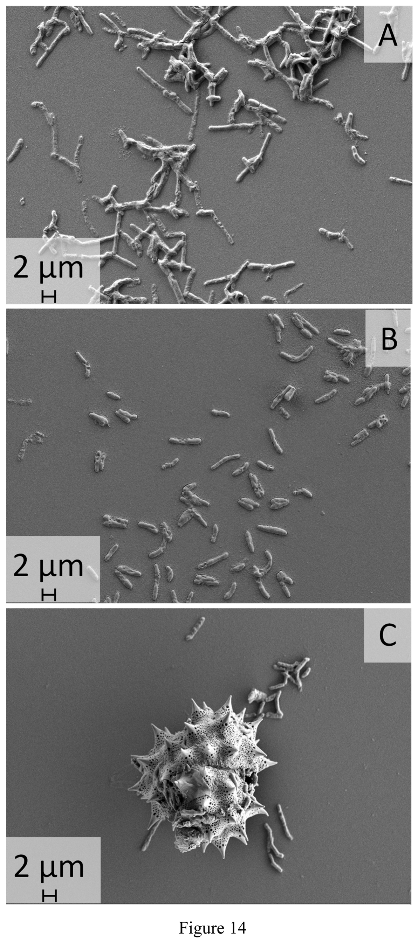

Figure 14. SEM images of *M. smegmatis* after treatment with protamine nanosystems with rifabutin (A), with chitosan nanosystems with rifabutin (B), and with microcapsules according to the invention, wherein the nanosystems comprise chitosan with rifabutin (C).

**Detailed Description of the Invention**

Definitions

The term "pharmaceutically active compound" herein is a synonym of "pharmaceutically active agent" or "pharmaceutically active ingredient" and includes not only those substances capable of a pharmaceutical effect, but also any acid, base, salt, or polymorphic form thereof or other derivatives that are capable of a pharmaceutical effect when absorbed by the body.
The term "surfactant" encompasses any derivative thereof capable of being converted into surfactant following pulmonary application.
A "pharmaceutically acceptable" component is understood to mean a component which is not undesirable from the biological or any other viewpoint, for example, the component can be incorporated in a pharmaceutical formulation of the invention and delivered to a patient as described herein without causing an undesired biological effect or interacting adversely with any of the other components of the formulation in which it is contained. Where the term "pharmaceutically acceptable" is used to refer to an excipient or carrier, it means that said excipient or carrier meets the required standards of toxicological and manufacturing tests according to the FDA and/or the European Pharmacopeia.
Herein, "mass median aerodynamic diameter" or "MMAD", or simply "aerodynamic diameter" has the common meaning in the art. It refers to the median aerodynamic size of a plurality of microparticles, typically in a polydispersed population. The "aerodynamic diameter" may be the diameter of a unit density sphere having the same sedimentation speed, generally in the air, as a powder and is therefore a useful way of characterizing an aerosol powder or another dispersed particle or particle formulation in terms of sedimentation. The aerodynamic diameter encompasses the shape, density, and physical size of the microparticle. Herein, MMAD refers to the mass median aerodynamic diameter of the microparticle, or the particle size distribution of microparticles in aerosol, determined by cascade impact. Cascade impaction devices include a series of stages with decreasing pore size. The stages trap particles within a moving jet passing through the impactor. The amount of particulate material (having particle sizes within a defined size range) that is trapped in each stage can be determined by washing the stage and measuring the amount of eluted material. An example of a cascade impactor is the Andersen Cascade Impactor.

Microparticles of the Invention

The microparticles of the present invention comprise purified pollen particles (comprising an intine and exine layer) and a nanosystem comprising the drug. In the context of the present invention, the purified pollen particles are referred to as capsules. In the present invention, the capsules comprise a pharmaceutically acceptable excipient on their outer surface.

In a particular embodiment, the microparticles have an aerodynamic diameter (MMAD) of less than 15 $\mu$m, preferably less than 10 $\mu$m, preferably between 1 and 10 $\mu$m, more preferably between 2 and 10 $\mu$m, even more preferably between 3 and 9 $\mu$m. In a more preferred embodiment, the microparticles have an aerodynamic diameter (MMAD) of between 5 and 10 $\mu$m, even more preferably between 6 and 9 $\mu$m. The MMAD is determined by cascade impact, as explained herein.

Pollen capsules

The microparticles of the present invention comprise purified pollen particles, which are referred to herein as "pollen capsules" or simply "capsules". These capsules are the result of purifying pollen particles with organic and acidic solvents so as to remove their inside (sporoplasm) and their outermost layer (pollenkitt). The purification method is described in the section of examples. Therefore, in this disclosure, the pollen capsules are also identified as "purified pollen capsules".

Preferably, the purification method completely or partially empties the sporoplasm. Therefore, in a particular embodiment, the pollen capsules comprise less than 90%, preferably less than 80%, 70%, 60%, 50%, 40%, 30%, 20%, even more preferably less than 10% by weight of cytoplasm, with respect to the original weight of the cytoplasm. In a preferred embodiment, the pollen capsules do not comprise sporoplasm.

The pollen capsules thus comprise a double-layer shell, with the inner layer being intine and the outer layer being exine. Furthermore, said purification may not be complete purification, and may still partially retain the lipid layer.

In a particular embodiment, the pollen capsules retain less than 50%, preferably less than 40%, preferably less than 20%, preferably less than 10%, even more preferably less than 5% by weight of the lipid layer, with respect to the original weight of the lipid layer.

In a particular embodiment, the pollen capsule is devoid of its lipid layer (pollenkitt). The absence of pollenkitt allows the shell of the capsules to be porous, enabling the penetration of the nanosystems into the capsules. Without wishing to be bound by any theory in particular, it is considered that the pollenkitt fills the nanopores of the capsules which are present naturally in their structure, as well as their larger opening. Both the nanopores and the openings of the capsules vary in number and size, and may be present or absent depending on the type of pollen used. Pollenkitt removal leads to the opening of the nanopores, enabling the encapsulation of certain nanosystems depending on their size at the exine level. However, for the inner encapsulation of the nanosystems, in addition to pollenkitt removal, there is a need to carry out sporoplasm removal.

Therefore, in another particular embodiment, the pollen capsule is devoid of sporoplasm.

Pharmaceutically acceptable excipients

The microparticles of the invention comprise one or more pharmaceutically acceptable excipients which are suitable for pulmonary delivery.

In the context of the present invention, the pharmaceutically acceptable excipients, once coating the microparticle, should improve its aerosolization and/or dispersion, compared to uncoated microparticles. These excipients are used in the present invention to facilitate the flow of the microparticles inside the respiratory tract, particularly to facilitate microparticle vectorization towards the tracheobronchial and pulmonary regions. Preferably, the pharmaceutically acceptable excipients coating the microparticles of the invention improve their flow properties and the aerodynamic diameter of the microparticles. These properties allow the microparticles to readily reach the regions of interest, such as the tracheobronchial region, and preferably the pulmonary region, more preferably the alveoli.

In particular, the excipient materials can function normally to even further improve the physical and chemical stability of the active agent, minimize residual moisture content, and prevent moisture uptake, and to improve particle size, degree of aggregation, particle surface properties (i.e., roughness), ease of inhalation, and directing the particles to the lung.

In a particular embodiment, the pharmaceutically acceptable excipients constitute the outermost layer of the microparticle of the invention.

In a particular embodiment, the pharmaceutically acceptable excipient reduces the mass median aerodynamic diameter of the microparticle by at least 10%, when compared to an identical microparticle but without a pharmaceutically acceptable excipient, determined with an Andersen Cascade Impactor. Preferably, the pharmaceutically acceptable excipient reduces the mass median aerodynamic diameter of the microparticle by at least 20%, when compared to an identical microparticle but without a pharmaceutically acceptable excipient, determined with an Andersen Cascade Impactor.

In a particular embodiment, the pharmaceutically acceptable excipients are selected from the group consisting of amino acids, peptides, proteins, polymers, polyols, carbohydrates, fatty acids, fatty acid salts, phospholipids, and combinations thereof.

Examples of amino acids include alanine, glycine, arginine, betaine, histidine, glutamic acid, aspartic acid, cysteine, lysine, leucine, isoleucine, valine, methionine, phenylalanine, aspartame, tyrosine, tryptophan, proline, and the like.

Examples of peptides include dimers, trimers, tetramers, and pentamers comprising one or more hydrophobic amino acid components such as those which have been described above.

Examples of proteins include albumins such as human serum albumin (HSA), recombinant human albumin (rHA), gelatin, casein, hemoglobin, and the like.

Examples of polymers include polyvinylpyrrolidones, derivatized celluloses such as hydroxymethylcellulose, hydroxyethylcellulose, and hydroxypropylmethylcellulose, Ficolls (a polymer sugar), hydroxyethyl starch, dextrates (for example, cyclodextrins, such as 2-hydroxypropyl-β-cyclodextrin and sulfobutylether-β-cyclodextrin, polyethylene glycols, and pectin.

Examples of carbohydrates are sugars, derivatized sugars such as alditols, aldonic acids, esterified sugars, and sugar polymers. In particular, carbohydrates suitable for use in the invention include monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches and the like; and alditols, such as mannitol, xylitol, maltitol, lactitol, xylitol sorbitol (glucitol), pyranosyl sorbitol, myo-inositol, and the like.

Preferred examples of fatty acids and/or fatty acid salts are those fatty acids endogenous to the lung, preferably stearic acid, and even more preferably magnesium stearate.

In a preferred embodiment, the pharmaceutically acceptable excipients are selected from the group consisting of mannitol, leucine, trehalose, lactose, albumin, dipalmitoylphosphatidylcholine (DPPC), and combinations thereof. Preferably, the pharmaceutically acceptable excipients are selected from the group consisting of mannitol, leucine, and combinations thereof.

These excipients coat the microparticles in amounts comprised between 10% and 95% by weight, preferably between 20% and 80%, and more preferably between 30% and 70% by weight, with respect to the weight of the uncoated microparticle of the invention. In an even more preferred embodiment, the excipients are present as a coating of the microparticles in amounts comprised between 40% and 60% by weight with respect to the weight of the uncoated microparticle of the invention.

In a particular embodiment, the excipients are adhered to the surface of the microparticles. Preferably, the adhesion is between the excipients and the exine layer of the pollen capsule.

In a preferred embodiment, the excipients are adhered to the surface of the microparticles by means of Van der Waals forces, preferably to the exine layer.

Nanosystems

The nanosystems of the present invention comprise a pharmaceutically active compound and are encapsulated in the pollen capsule. The nanosystems must be stables in tissues and biological fluids that are particularly related with the respiratory tract, particularly the lung.

In a particular embodiment, the nanosystems comprise polysaccharides, polyamino acids, or polypeptides, for example chitosan, polyarginine, and/or protamine.

In a preferred embodiment, the polyamino acids are selected from the group consisting of polyaspartic acid, polyglutamic acid, or polylysine.

In another preferred embodiment, the polypeptides are selected from the group consisting of cell-penetrating peptides (CPPs), oligopeptides, low molecular weight protamine (LMWP). Preferably, the oligopeptides are rich in arginine, such as octaarginines.

In another preferred embodiment, the polysaccharides are selected from the group consisting of chitosan, glycoproteins such as polysialic acid or synthetic polymers such as lactic-co-glycolic acid (PLGA) or polylactic acid (PLA). In a preferred embodiment, the nanosystem comprises chitosan.

In a preferred embodiment, the nanosystem comprises chitosan or protamine. In another preferred embodiment of the preceding one, the nanosystem comprises chitosan or protamine and a pharmaceutically active compound.

Protamine is of special interest in the present invention due to its capacity to interact with cells and take in drugs. Without wishing to be bound by any theory in particular, the inventors consider that the advantageous use of protamine is due to the repetitive arginine sequence present in its structure, providing it with an activity of efficiently translocating through biological membranes, including alveolar cells. Therefore, in a preferred embodiment, the nanosystems of the microparticles of the invention comprise protamine and a pharmaceutically active compound. Furthermore, and without wishing to be bound by any theory in particular, the inventors consider that protamine forms a protein layer which encapsulates the pharmaceutically active compound.

In a particular embodiment, the nanosystem further comprises an oily component. In the context of the present invention, the term "oily" should be understood as a synonym of "oleaginous". Preferably, the nanosystem comprises a protamine layer which encapsulates the pharmaceutically active compound and an oily component. The oily component can be a medium- or long-chain monoglyceride, diglyceride, and/or triglyceride. In a particular embodiment, the oily component is selected from the group consisting of peanut oil, cotton seed oil, olive oil, castor oil, soybean oil, safflower oil, geranium oil, palm oil, alpha-tocopherol (vitamin E), oleic acid, linoleic acid, isopropyl myristate, squalene, Miglyol®, Labrafil®, Labrafac®, Peceol® , Maisine® , caprylic/capric triglyceride, linoleoyl macrogol-6 glycerides (corn oil PEG-6 esters), glyceryl oleate, glyceryl linoleate, glycerol monooleate, and combinations thereof. Miglyol® is the commercial name of a product containing a mixture of decanoyl and octanoyl (capric/caprylic acid) glycerides. Labrafil® is the brand of a commercial product containing oleoyl macrogol-6 glycerides. Labrafac® is the brand of a commercial product containing medium-chain fatty acid triglycerides, known as caprylic/capric triglyceride (HLB 1). Peceol® is the brand of a commercial product containing glycerol monooleates (type 40). Maisine® is the brand of a commercial product containing glycerol monolinoleate. In a preferred embodiment, the oily compound is selected from the group consisting of squalene oil, flavoring oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicaprylate ketones, Cerafilos®, decyl oleate, alkyl $C_{12}$-$C_{15}$ lactates, cetyl lactate, lauryl lactate, isostearyl neopentanoate, myristyl lactate, isocetyl stearoyl stearate, octyldodecyl stearoyl stearate, hydrocarbon oils, isoparaffin, liquid paraffins, isododecane, vaseline, argan oil, rapeseed oil, chili oil, coconut oil, corn oil, cotton seed oil, linseed oil, grape seed oil, mustard oil, olive oil, palm oil, fractionated palm oil, groundnut oil, castor oil, pine nut oil, poppyseed oil, pumpkin seed oil, rice bran oil, safflower oil, tea oil, truffle oil, plant oil, apricot oil, jojoba oil, macadamia oil, wheat germ oil, almond oil, soybean oil, sesame oil, hazelnut oil, sunflower oil, hemp oil, candlenut oil, avocado oil, nut oil, fish oil, berry oil, Jamaican pepper oil, juniper oil, seed oil, almond seed oil, anise oil, celery seed oil, cumin seed oil, nutmeg seed oil, basil leaf oil, bay leaf oil, cinnamon leaf oil, sage leaf oil, eucalyptus leaf oil, lemon leaf oil, tea tree oil, oregano oil, patchouli leaf oil, mint leaf oil, pine needle oil, rosemary leaf oil, spearmint oil, tea tree leaf oil, thyme leaf oil, Canada tea leaf oil, flower oil, chamomile oil, sage oil, clove oil, geranium flower oil, hyssop flower oil, jasmine flower oil, lavender flower oil, mauka flower oil, marjoram flower oil, orange flower oil, rose flower oil, ylang-ylang flower oil, bark oil, cassia bark oil, cinnamon bark oil, sassafras bark oil, wood oil, camphor wood oil, cedar wood oil, rose stem oil, sandalwood oil, ginger wood oil, resin oil, castor oil, myrrh oil, peel oil, bergamo peel oil, grapefruit peel oil, lemon peel oil, lime peel oil, orange peel oil, tangerine peel oil, root oil, valerian oil, oleic acid, linoleic acid, oleic alcohol, isostearilic alcohol, ethyl oleate, Miglyol®, Labrafil®, Labrafac®, Rylo®, Peceol®, and Maisine®, synthetic or semi-synthetic derivatives thereof and combinations thereof. In a more preferred embodiment, the oily component is selected from the group consisting of peanut oil, cotton seed oil, olive oil, castor oil, soybean oil, safflower oil, palm oil, otocopherol (vitamin E), isopropyl myristate, squalene, Miglyol®, Labrafil®, Labrafac® , Peceol®, and Maisine® or mixtures thereof. More preferably, the oils are Miglyol®, squalene, or a-tocopherol.

In a preferred embodiment, the oily component is selected from the group consisting of oleic acid, caprylic/capric triglyceride (such as Miglyol®812), squalene, alpha-tocopherol, and combinations thereof.

In another particular embodiment, the nanosystem further comprises a surfactant, preferably lung tissue-compatible surfactants. In a particular embodiment, the surfactant is characterized by a hydrophilic-lipophilic balance (HLB) of more than 3, preferably more than 8. In a preferred embodiment, the surfactant is selected from the group consisting of polyoxyethylene sorbitan monooleate (polysorbate 80; Tween 80 ®; HLB 15), polyoxyethylene sorbitan monolaurate (polysorbate 20; Tween 20 ®; HLB 16.7), polyoxyethylene sorbitan monostearate (Tween ® 60, HLB 14.9 and Tween 61 ®; HLB 9.6), polyoxyethylene sorbitan monooleate (Tween 81®; HLB 10), polyoxyethylene sorbitan tristearate (Tween 65®; HLB 10.5), polyoxyethylene sorbitan trioleate (Tween 85®; HLB 11), polyoxyethylene sorbitan monolaurate (Tween® 20, HLB 16.7 and Tween 21 ® HLB 13.3); PEGylated fatty acid esters and mixtures with PEG, polyethylene glycol monostearate (HLB 11.6), polyethylene glycol stearate, polyethylene glycol stearate 40 (HLB 17), polyethylene glycol dilaurate 400 (HLB 9.7), polyethylene glycol monopalmitate (HLB 11.6), polyethylene glycol stearate, polyethylene glycol stearate 40 (HLB 16.9) polyethylene glycol stearate 100 (HLB 18.8), Solutol HS15® (HLB 15), polyethylene glycol 15-hydroxystearate (HLB 14-16), D-alpha-tocopherol polyethylene glycol succinate (TPGS; HLB 13.2), triethanolammonium oleate (HLB 12), sodium oleate (HLB 18), sodium cholate (HLB 18), sodium deoxycholate (HLB 16), sodium lauryl sulfate (HLB 40), sodium glycocholate (HLB 16-18), triethanolamine oleate (HLB 12), gum tragacanth (HLB 11.9), and sodium dodecylsulfate (HLB 40); Poloxamer 124 (HLB 16), Poloxamer 188 (HLB 29), Poloxamer 237 (HLB 29), Poloxamer 238 (HLB 28), Poloxamer 278 (HLB 28), Poloxamer 338 (HLB 27) and Poloxamer 407 (HLB 22), sorbitan monooleate (Span® 80, HLB 4.3), sorbitan monolaurate (Span® 20, HLB 8.6), sorbitan monostearate (Span® 60, HLB 4.7), sorbitan trioleate (Span® 85, HLB 1.8), sorbitan sesquioleate (Span® 83, HLB 3.7), sorbitan monopalmitate (Span® 40, HLB 6.7), sorbitan isostearate (Span® 120, HLB 4.7), polyoxyethylene sorbitan monopalmitate (Tween® 40, HLB 15.6), and combinations thereof.

In a preferred embodiment, the nanosystem comprises a surfactant selected from the group consisting of Solutol® HS 15, Tween® 20, Tween® 80, Span® 80, Labrasol®, Poloxamer 188, sodium glycocholate, and combinations thereof. In an even more preferred embodiment, the surfactant is not a phospholipid.

In another particular embodiment, the nanosystem further comprises a stabilizing agent, preferably on the outer surface of the nanosystem. The optional presence of a stabilizing agent serves to prevent the aggregation of the nanosystems. In a preferred embodiment, the stabilizing agent is selected from the group consisting of lecithin, polyvinyl alcohol, Kolliphor®, polyethylene glycol stearate (PEGst), and mixtures thereof. Examples of Kolliphor® are Kolliphor PS 20, Kolliphor PS 60, or Kolliphor PS 80 (CAS numbers 9005-64-5, 9005-67-8, and 9005-65-6, respectively), Kolliphor EL (CAS number 61791-12-6), Kolliphor SLS (CAS number 151-21-3), or Kolliphor P188 (CAS number 9003-11-6).

Therefore, in a particular embodiment, the nanosystem comprises an outer layer comprising the pharmaceutically active compound, optionally an oily component, and is optionally coated with a stabilizing agent.

In a preferred embodiment, the nanosystem comprises an outer protamine layer comprising an emulsion with at least one pharmaceutically active compound and an oily component, optionally Miglyol®812, and is coated with a stabilizing agent, preferably polyethylene glycol stearate.

In a particular embodiment, the nanosystems of the microparticles of the invention have a hydrodynamic diameter comprised between 100 and 500 nm, preferably between 100 and 300 nm, more preferably between 150 and 250 nm, measured by means of dynamic light scattering (DLS).

In a particular embodiment, the nanosystems of the microparticles of the invention have a positive, negative, or neutral surface charge. In another particular embodiment, the nanosystems of the microparticles of the invention have a positive surface charge. In another particular embodiment, the nanosystems of the microparticles of the invention have a negative surface charge. In another particular embodiment, the nanosystems of the microparticles of the invention have a neutral surface charge.

Pharmaceutically active compound

The microparticles of the present invention comprise a pharmaceutically active compound comprised in a nanosystem which is in turn comprised in pollen capsules for pulmonary delivery. Compared to the oral route, the pulmonary route allows rapid action and higher bioavailability due to the large surface and high alveolar vascularization (70-140 m$^2$). In the present invention, the pharmaceutically active compound must be suitable for pulmonary delivery.

In a particular embodiment, the pharmaceutically active compound is an antibiotic, preferably for the treatment of tuberculosis. Preferably, the compound is selected from isoniazid, rifampicin, ethambutol, pyrazinamide, kanamycin, moxifloxacin, levofloxacin, or rifabutin. Rifabutin is a rifamycin derivative and a primarily bactericidal antibiotic used for treating tuberculosis.

In a particular embodiment, the pharmaceutically active compound is selected from the group consisting of insulin and rifabutin.

In a preferred embodiment, the pharmaceutically active compound is rifabutin.

In another preferred embodiment, the pharmaceutically active compound is insulin.

In a particular embodiment, the pharmaceutically active compound is in a concentration comprised between 0.5% and 20% by weight, with respect to the weight of the nanosystem. Preferably, the pharmaceutically active compound is in a concentration comprised between 0.5% and 10% by weight, more preferably between 0.5% and 5% by weight, even more preferably between 0.5% and 5% by weight, with respect to the weight of the nanosystem. In this context, a concentration of, for example, 2% by weight with respect to the weight of the nanosystem means that there are 2 mg of active compound per every 100 mg of nanosystem.

In a particular embodiment, the pharmaceutically active compound is in a concentration comprised between 0.5% and 20% by weight, with respect to the total weight of the capsules. Preferably, the pharmaceutically active compound is in a concentration comprised between 0.5% and 10% by weight, more preferably between 0.5% and 5% by weight, even more preferably between 0.5% and 5% by weight, with respect to the total weight of the capsules. In this context, a concentration of, for example, 2% by weight with respect to the weight of the nanosystem means that there are 2 mg of active compound per every 100 mg of capsules.

Method of obtaining

According to the second aspect, the method of the invention is a method for the preparation of the microparticle of the first aspect, i.e., the microparticle of the invention. The method comprises the following stages:

(a) a washing stage which comprises washing a pollen particle;
(b) a stage which comprises treating the pollen particle obtained in the preceding stage with an acidic medium to obtain hollow purified pollen capsules;
(c) a stage which comprises incubating the hollow capsules obtained in the preceding stage with a nanosystem comprising a pharmaceutically active compound; and
(d) a stage which comprises coating the resulting capsules with a pharmaceutically acceptable excipient.

Washing stage (a) allows the removal of pollenkitt, wherein pollenkitt is the lipid layer that coats pollen particles. In a particular embodiment, washing allows the partial removal of pollenkitt, preferably the complete removal of pollenkitt. Stage (a) comprises washing the pollen particle in an aqueous medium, in an organic medium, in an aqueous medium followed by an organic medium, or in an organic medium followed by an aqueous medium.

In a particular embodiment, stage (a) comprises the step of washing the pollen particles with water at a temperature between 20°C and 60°C, preferably between 20°C and 50°C, more preferably between 20°C and 45°C, even more preferably between 20°C and 40°C. This step preferably comprises suspending the particles in water, and can be carried out under stirring, preferably for a time comprised between 1 and 60 minutes, preferably between 1 and 20 minutes, more preferably between 1 and 10 minutes, and even more preferably between 2 and 10 minutes. In a particular embodiment, stage (a) comprises the additional step of recovering washed particles, preferably by means of filtration. These preceding steps can be repeated if necessary, for example, by subjecting the particles to washing several times with water under stirring and recovering by means of filtration. Finally, stage (a) may include the last step of drying the washed particles.

In a particular embodiment, stage (a) comprises the step of washing the particles with an organic solvent selected from the group consisting of cyclic or linear hydrocarbons such as, for example, of formula $C_nH_{n+2}$ (non-cyclic, linear, or branched) or $C_nH_{2n}$ (cyclic), with 5 to 20 carbon atoms (n=5-20). Examples which can be used in the present invention are pentane, hexane, heptane, octane, nonane, decane, cyclopentane, or cyclohexane, or mixtures thereof, preferably, cyclohexane. The organic solvent can also be an alcohol with, for example, 1 to 12 carbon atoms, for example, 1 to 4 carbon atoms. Examples are methanol, ethanol, propanol, butanol, or mixtures thereof. Non-limiting examples also include halogenated hydrocarbons, i.e., hydrocarbons such as those defined above in this paragraph, but in which at least one of the hydrogen atoms has been substituted by a halogen (fluorine, chlorine, bromine, or iodine, preferably chlorine). The halogenated hydrocarbon used in the present invention may have the formula $C_nH_{2n+2-z}X_z$ (non-cyclic, linear, or branched) or $C_nH_{2n-z}X_z$ (cyclic), wherein z is an integer (equal to or less than 2n+2 or 2n, as the case may be) and X is fluorine, chlorine, bromine, or iodine, preferably chlorine. Examples of solvents of this type are dichloromethane and chloroform. Washing can also be performed by using mixtures of organic solvents in different proportions, for example, by using a mixture of halogenated hydrocarbon:alcohol in proportions comprised between 1:20 and 20:1. An example can be the mixture of chloroform and methanol. Other organic solvents which can be used in the present invention are those including a carbonyl or ester group and having a low molecular weight, for example, below 250 Da. Extended examples of these solvents are acetone or ethyl acetate. Therefore, in a preferred embodiment, stage (a) comprises washing with an organic solvent selected from acetone, cyclohexane, chloroform, dichloromethane, methanol, and mixtures thereof. Like in the case of washing with water, more than one wash can be performed with organic solvents. It is also preferred to perform washing with an organic solvent at low or moderate temperatures, for example, at a temperature comprised between 15°C and 60°C, preferably between 20°C and 45°C. Washing with an organic solvent preferably comprises homogenization of the suspension for a time comprised between 1 and 60 minutes, preferably between 1 and 20 minutes, more preferably between 1 and 10 minutes, and even more preferably between 2 and 10 minutes. In a preferred embodiment, the particles in suspension in an organic medium are vacuum filtered and dried, optionally at a temperature comprised between 25°C and 80°C, for example, at 60°C. These steps of stage (a) can be repeated, for example, with different organic solvents.

The acid treatment stage (b) allows completely or partially emptying the pollen particle of its cytoplasm, i.e., the sporoplasm. In a particular embodiment, acid treatment allows the partial removal of the cytoplasm. Preferably, it allows the complete removal of the cytoplasm. In a particular embodiment, stage (b) comprises treatment with phosphoric acid. In another particular embodiment, stage (b) comprises acid treatment for a time period comprised between 1 and 14 hours, preferably between 1 and 10 hours, more preferably between 1 and 6 hours, even more preferably between 3 and 6 hours. The treatment duration must be such that the pollen capsules do not lose their intine layer. Therefore, in a preferred embodiment, stage (b) is carried out with phosphoric acid for a period comprised between 1 and 6 hours. In a preferred embodiment, stage (b) is carried out at a temperature greater than room temperature, preferably between 30°C and 100°C, more preferably between 50 and 80°C, even more preferably between 60 and 80°C.

Stage (c) which comprises incubating the hollow capsules obtained in the preceding stage with a nanosystem comprising a pharmaceutically active compound allows encapsulating the nanosystems inside said capsules by means of a simple incubation process. Said nanosystems are those explained in detail herein in the section dedicated to nanosystems and exemplified in a non-limiting manner in the section of examples. In a particular embodiment, stage (c) is carried out without any organic solvent, being able to be carried out in the presence of water. In another particular embodiment, stage (c) is carried out under stirring, preferably under conditions with rotational movement. In another particular embodiment, stage (c) is carried out at a pressure less than the atmospheric pressure. Preferably, the pressure is comprised between 0.1 and 500 mbar, preferably between 1 and 250 mbar, more preferably between 1 and 100 mbar, even more preferably between 10 and 100 mbar. In another particular embodiment, stage (c) is carried out at a temperature equal to or greater than room temperature, preferably greater than 30°C and preferably less than 50°C. Stage (c) can be carried out for a time period comprised between 1 and 1000 minutes, preferably between 1 and 120 minutes, more preferably between 1 and 60 minutes, and even more preferably between 20 and 40 minutes.

In a particular embodiment of the method of the invention, the nanosystems are prepared by means of a solvent displacement technique. Particularly, the nanosystems can be prepared by contacting the ingredients required for obtaining a nanoemulsion. In a preferred embodiment, the pharmaceutically active compound is mixed with an oily component and a surfactant in an organic solvent, and this mixture is contacted with polysaccharides, polyamino acids, or polypeptides in an aqueous solvent. Once the emulsion is formed, the organic part is evaporated and the nanosystems (which are not water-soluble) can be recovered by means of, for example, centrifugation. In a more preferred embodiment, the pharmaceutically active compound, the oily component, and/or the surfactant are selected from those which are described above as being preferred, in the section dedicated to the nanosystems. The solvent to be used will depend on the nanosystem, as long as it allows obtaining same. In a more preferred embodiment, the organic solvent is selected from acetone or ethanol.

Stage (d) which comprises the step of coating the capsules which comprise therein the nanosystems resulting from the preceding stage with a pharmaceutically acceptable excipient allows obtaining the microparticles of the invention which comprise a pharmaceutically acceptable excipient coated on the purified pollen capsules comprising an intine layer and an exine layer and a nanosystem therein. In a particular embodiment, stage (d) comprises contacting the capsules of the preceding stage with a pharmaceutically acceptable excipient. Preferably, coating is carried out by means of the dry mechanical coating technique (slid mixture), spray drying, or lyophilization. More preferably, it is carried out by means of lyophilization. In a particular embodiment, coating is carried out for a time period comprised between 1 minute and 48 hours, preferably between 1 minute and 30 hours. In a preferred embodiment, if coating is carried out by means of lyophilization, the duration is comprised between 1 hour and 30 hours, preferably between 10 and 30 hours, more preferably between 20 and 30 hours.

Obtainable microparticle which can be obtained according to the method

A third aspect of the present invention relates to a microparticle for pulmonary release, characterized in that it is obtained according to the method of the second aspect. This aspect defines the microparticle in its broadest sense as described herein, as well as the microparticle comprising any of the particular and/or preferred embodiments described herein. Likewise, for the same reasons, the obtainable microparticle which can be obtained according to the method of the invention is considered to include the method of obtaining described in a broader sense, as well as the method of obtaining which comprises any of the particular and/or preferred embodiments described herein.

Pharmaceutical composition

A fourth aspect of the present invention relates to a pharmaceutical composition, characterized in that it comprises a microparticle according to the first or third aspect, and a pharmaceutically acceptable excipient.

In this context, the pharmaceutically acceptable excipient is not part of the microparticle and is added once the microparticle is prepared for the purpose of improving certain property of interest for the pulmonary delivery route such as, for example, dispersibility or moisture protection, or as diluent. Therefore, the excipient is selected from pulmonary delivery-compatible excipients. In a particular embodiment, the excipient is selected from surfactants, amino acids, sugars, lipids, phytosterols, and/or biodegradable polymers.

Examples of amino acids are leucine and/or trileucine. Examples of sugars are lactose, alginate, mannitol, trehalose, and/or pullulan. Examples of lipids are magnesium or calcium stearate, and/or phospholipids. Examples of biodegradable polymers are microcrystalline cellulose, the poly(lactic-co-glycolic acid) (PLGA), and/or chitosan.

Medical uses

A fifth aspect of the present invention relates to a microparticle according to the first or third aspect, or a pharmaceutical composition according to the fourth aspect, for use as a medicinal product.

A sixth aspect of the present invention relates to a microparticle according to the first or third aspect, or a pharmaceutical composition according to the fourth aspect, for use in the treatment of lung-related diseases.

In a preferred embodiment of the sixth aspect of the invention, the diseases are selected from the group consisting of asbestosis, asthma, bronchiectasis, bronchitis, bronchiolitis, chronic cough, chronic obstructive pulmonary disease (COPD), cystic fibrosis, idiopathic pulmonary fibrosis (IPF), interstitial pulmonary disease (IPD), lung cancer, pleuritis, pneumonia, pulmonary embolism, pulmonary fibrosis, pulmonary hypertension, sarcoidosis, and viral or bacterial diseases. In a preferred embodiment, the viral diseases are those caused by SARS, MERS, hantaviridae, influenza virus, rhinovirus, or human respiratory syncytial virus. In a preferred embodiment, the bacterial diseases are selected from the group consisting of whooping cough, bacterial pneumonia, and tuberculosis.

In a preferred embodiment of the sixth aspect of the invention, the disease is tuberculosis.

The sixth aspect of the present invention can likewise be defined in an alternative manner. In that sense, this aspect relates to the use of the microparticles of the invention, or of the obtainable microparticles which can be obtained according to the method of the invention, or of the pharmaceutical composition, for manufacturing a medicinal product for the treatment of lung-related diseases. A second alternative way of defining this aspect is a method for the treatment of lung-related diseases, which comprises delivering to a subject in need thereof an effective amount of the microparticles of the invention, or of the obtainable microparticles which can be obtained according to the method of the invention, or of the pharmaceutical composition.

Inhalers

In a particular embodiment, the microparticle of the invention is delivered by means of a pulmonary delivery system, preferably selected from nebulizers, pressurized metered-dose inhalers (pMDIs), or dry powder inhalers (DPIs). In a preferred embodiment, it is delivered using a dry powder inhaler.

In a particular embodiment of the sixth aspect of the invention, treatment is performed through delivery by means of a nebulizer, a pressurized metered-dose inhaler, or a dry powder inhaler, preferably a dry powder inhaler.

Experimental part

The examples of the present disclosure describe the synthesis of purified pollen capsules comprising an intine layer and an exine layer (Example 1), the method of coating the capsules with a pharmaceutically acceptable excipient (Example 2), the synthesis of nanosystems comprising protamine and the drug rifabutin (Example 3), and the encapsulation of the nanosystems in pollen capsules (Example 4).

The other examples of the present disclosure also describe the characterization of the capsules and nanosystems by means of structural, thermoanalytical, and aerodynamic analyses. Rifabutin release profile was evaluated in a simulated lung medium and microparticle distribution was evaluated in an *in vitro* lung model. The examples demonstrate that coating the pollen capsules improve their distribution in the lung, with successful rifabutin encapsulation and release. These examples serve to illustrate the embodiments of the invention, but they should not be considered limiting in any case.

Materials and Methods

Rifabutin was acquired from *Chemos GmbH* (Germany). Low molecular weight protamine sulfate (5 kDa) was acquired from *Yuki Gosei Kogyo, Ltd* (Japan). The polyethylene glycol stearate 40 (PEGst 40) was provided by *Croda Chemicals Europe Ltd.* (United Kingdom) and sodium glycocholate (SGC) was acquired from *Dextra laboratories Ltd.* (United Kingdom). Miglyol® 812 (caprylic/capric/succinic triglyceride) was obtained from *IOI Oleo GmbH* (Germany). The 1,1'-dioctadecyl-3,3,3',3'-tetramethylindodicarbocyanine 4-chlorobenzenesulfonate (DiD) salt was acquired from *Thermo Fisher Scientific* (USA). The 3-(4,5-dimethyl-2-thiazolyl)-2,5-diphenyl-2H-tetrazolium bromide (MTT) salt, fetal bovine serum (FBS), and PSG (Penicillin-Streptomycin-Glutamine) were purchased from *Sigma-Aldrich. Dulbecco's Modified Eagle* (DMEM) medium was obtained from *GIBCO®* (*Thermo Fisher Scientific,* Spain). Cell line *Raw 264.7* was obtained from *ATCC* (Manassas, VA, USA). Crude sunflower (*Helianthus annuus*) pollen was purchased from *Pharmallerga* (Lisov, Czech Republic) and dry chamomile flowers (*Matricaria chamomilla*) were purchased from *Soria Natural* (Soria, Spain). D-mannitol was purchased from *Molar Chemical KFT* (Hungary). L-leucine was purchased from *AppliChem GmbH* (Germany). The other reagents were of analytical grade and used without additional purification.

Analytical measurements

Samples were characterized by FT-IR (Thermo Nicolet AVATAR 330, Waltham, MA, USA) with a resolution of 4 cm$^{-1}$ in the wavenumber range of 400-4000 cm$^{-1}$, and FT-IR spectra were analyzed using the Spectragryph software v1.2.16.1.

Samples were characterized by differential scanning calorimetry (DSC) using a DSC Mettler-Toledo 821e equipment (Mettler-Toledo GmbH, GieBen, Germany) in the temperature range of 30 to 450°C with constant argon purge, and spectra were analyzed with the STARe V9.1 thermal analysis program (Mettler Inc., Schwerzenbach, Switzerland).

Sample crystallinity was determined by means of powder sample X-ray diffraction (XRPD) using the diffractometer from Bruker AXS GmbH, Karlsruhe, Germany. The conditions were: Cu target, Ni filter, voltage of 40 kV, amperage of 40 mA, time constant of 0.1°/min, and angular step of 0.010° (interval 3-40°). Diffractograms were evaluated with the DIFFRACplus EVA diffraction software (Bruker AXS GmbH, Karlsruhe, Germany).

Sample morphology was determined by means of SEM (Hitachi S4700, Hitachi Scientific Ltd., Tokyo, Japan) after coating the samples with gold-palladium in an argon atmosphere.

Encapsulation was studied using confocal laser microscopy (Confocal Laser Scanning Microscopy, SP5 Leica AOBS-SP5, Leica Biosystems Nussloch GmbH). Fluorescence measurements were performed at the excitation wavelength of the DiD dye ($\lambda_{max}$=640 nm) and emission wavelength ($\lambda_{max}$= 675 nm), and natural pollen fluorophores were subjected to excitation at $\lambda_{max}$=405 nm, 492 nm, and 561 nm and collected with emission filters of $\lambda_{max}$= 414-479, 505-554, and 571-635 nm.

The statistical significance of the difference between the means was determined by means of GraphPad Prism 8 and OriginPro 9.0. All the data is shown as average $\pm$ standard deviation and one- and two-way variance analyses (ANOVA) were performed to compare various independent groups. Differences were considered statistically significant for $P<0.05$.

Example 1. Obtaining purified pollen capsules

Two types of pollen were used, both belonging to the *Asteraceae family:* sunflower (*H. annuus*) pollen with a geometric size of about 35 $\mu$m, and chamomile (*M. chamomilla*) pollen with a geometric size of about 20 $\mu$m.

*H. annuus* and *M. chamomilla* pollen grains were treated in several consecutive washing steps to remove the outer coating of sporopollenin (pollenkitt) and inner cellular components (sporoplasm) according to the method described in Ageitos, J.M., et al.; "Purification of Hollow Sporopollenin Microcapsules from Sunflower and Chamomile Pollen Grains"; Polymers (Basel), 2021, 13, 2094.

Following the methodology described in Ageitos JM *et al., H. annuus* pollen was treated with water to extract hydrophilic compounds. This step consisted of suspending 2 g of pollen particles in 40 mL of water, stirring for 5 minutes for a total of 5 repetitions, performing vacuum filtration, and drying at 60°C. Pollen washed with water was resuspended in 20 mL of acetone, homogenized for 5 minutes, and vacuum filtered. Pollen treated with acetone was resuspended in 20 mL of cyclohexane for 30 seconds to obtain defatted *H. annuus* capsules. To obtain hollow capsules, the defatted capsules were incubated with $H_3PO_4$ in a 1:10 proportion (w/v) at 70°C for 5 h.

*M. chamomilla* pollen was extracted from 15 grams of dry chamomile flowers by means of infusion with acetone for 30 minutes at 25°C. The infusion was filtered through stainless steel sieves equipped with 50 $\mu$m and 30 $\mu$m meshes, followed by a paper filter, using vacuum conditions. Luego, the process of extracting hydrophilic compounds with water was performed, as described above for *H. annuus.* In detail, this step consisted of suspending pollen particles extracted from 15 grams of dry flowers in 40 mL of water, stirring for 5 minutes for 5 repetitions, performing vacuum filtration, and drying at 60°C to obtain defatted *M. chamomilla* capsules. Finally, water-treated (defatted) pollen was incubated with $H_3PO_4$ in a 1:10 ratio (w/v) at 70°C for 5 h to obtain hollow *M. chamomilla* sporopollenin capsules.

Example 2. Coating of the purified pollen capsules.

The surface of the capsules obtained in Example 1 was coated with a pharmaceutically acceptable excipient (in this example, mannitol and/or leucine) using three techniques: (1) dry mechanical coating (solid mixture); (2) spray drying (or atomization); and (3) lyophilization.

In the case of solid mixture (1), the same amount of capsules and pharmaceutically acceptable excipient (proportion of 1:1 w/w) was mixed in a vortex for 5 minutes.

In the case of coating by means of spray drying (2), a suspension of pharmaceutically acceptable excipient and capsules (in a 80:20 proportion, respectively) was homogenized in water using a magnetic stirrer and inhalable powder was produced with a mini-spray dryer B-290 (*Büchi Labortechnik AG, Falwil,* Switzerland). The drying conditions were as follows: inlet temperature of 110°C, outlet temperature of 80°C (leucine) and 50°C (mannitol), suction unit capacity of 80%, feed pump flow rate of 20%, and a total solid content of 0.5% (5 mg/mL of capsules and excipients before drying).

In the case of coating by means of lyophilization (3), the capsules and the pharmaceutically acceptable excipients in water were lyophilized for 24 h using the Scanvac, Coolsafe 100-9 apparatus (*LaboGeneApS, Lynge,* Denmark) in a proportion of 1:1 (w/w) and a total solid content of 0.5% (5 mg/mL of capsules and excipients before lyophilization). Subsequent process yield determination was calculated by gravimetry. The resulting coated capsules were stored at room temperature until their subsequent characterization.

Example 3. Obtaining rifabutin and protamine nanosystem

Rifabutin-loaded protamine nanosystems were prepared by means of the solvent displacement technique following a method adapted from that described in Thwala, L.N.; Beloqui, A.; Csaba, N.S.; González-Touceda, D.; Tovar, S.; Dieguez, C.; Alonso, M.J.; Préat, V. The Interaction of Protamine Nanocapsules with the Intestinal Epithelium: A Mechanistic Approach. J. Control. Release 2016, 243, 109-120.

An oily core of 29.5 mg of Miglyol®812, 2.5 mg of SGC, 8 mg of PEGst-40, and rifabutin (5% theoretical drug with respect to the total weight of the nanosystem) was dissolved in acetone in a final volume of 2.5 mL and was poured into 5 mL of an aqueous phase of 1.5 mg/mL of protamine. The formulation was left under magnetic stirring at 1,000 rpm for 10 min. The organic solvent was evaporated under vacuum conditions (Rotavapor R-215, Büchi, Switzerland) and nanosystems were isolated by centrifugation (Hermle Labnet universal centrifuge Z323K, Labnet international) at 15,000 g a 15°C for 1 h. The isolated formulation was resuspended in ultrapure water.

To evaluate the distribution of the nanosystems in pollen capsules, the rifabutin-loaded protamine nanosystems were labelled by incorporating 50 $\mu$g of DiD in the organic phase before nanosystem formation. The rifabutin-loaded protamine nanosystems (with and without DiD) were analyzed by means of dynamic light scattering (Zetasizer Nano-ZSTM, Malvern Panalytical) and the analysis was performed in triplicate, at 25°C, and with a detection angle of 173°. Rifabutin encapsulation efficiency was quantified by means of an Acquity UPLC H-Class Plus system equipped with a C18 column (Waters® Symmetry of 1.7 $\mu$m, 2.1 x 50 mm) following a protocol developed by Rouco et al. [Rouco, H.; Diaz-Rodriguez, P.; Gaspar, D.P.; Gongalves, L.M.D.; Cuerva, M.; Remuñán-López, C.; Almeida, A.J.; Landin, M. Rifabutin-Loaded Nanostructured Lipid Carriers as a Tool in Oral Anti-Mycobacterial Treatment of Crohn's Disease. Nanomaterials 2020, 10, 1-18]. The results are described in Example 9.

Example 4. Obtaining microparticles for pulmonary release according to the invention

The encapsulation efficiency, loading capacity, and capsule content were studied in a fixed mass ratio of 1:1 (w/w). The loading (encapsulation) process was performed by contacting the nanosystems described in Example 3 with the capsules described in Example 1, under conditions with rotational movement (150 rpm) and vacuum (50 mbar) at 37°C for 30 minutes.

For the determination of non-encapsulated nanosystems, capsules were resuspended in water, centrifuged for 1 min at 15,000 g at room temperature, and the supernatant was collected using a syringe. Samples were filtered and lyophilized for 24 h, and nanosystem encapsulation was determined by gravimetry. Todos the experiments were performed in triplicate.

Once loaded with the nanosystems, the capsules were coated. To that end, capsules comprising the nanosystems were lyophilized for 24 h using the Scanvac, Coolsafe 100-9 apparatus in the presence of mannitol/leucine in a proportion of 1:0.5:0.5 (w/w, capsules:mannitol:leucine) in a final solid content of 0.5% (5 mg/mL of capsules and excipients before drying). The microparticles of the invention were stored at room temperature until use.

Example 5. Aerodynamic study of purified pollen capsules

Aerodynamic diameter is one of the main, if not the most important, factors when determining how particles are deposited in the different regions of the respiratory tract.

*In vitro* distribution profile was evaluated using an Andersen Cascade Impactor (AIC, *Copley Scientific Ltd., Nottingham,* United Kingdom). The methodology followed the requirements of the United States Pharmacopeia and Eu. Ph. 2.9.18. AIC separates particles according to their aerodynamic diameter by stages 1 to 6 (8.60, 6.50, 4.40, 3.20, 1.90, 1.20, 0.55, and 0.26 $\mu$m). A glass fiber filter (Pall Corporation, 1 $\mu$m, Mexico) was placed right below the last stage. The different stages were coated with a 1 + 99% w/w Span 80 + cyclohexane solution to prevent particle bouncing. 8 mg of sample were loaded in 4 hard gelatin capsules (transparent, size 3, Capsugel, Germany) and aerosolized using a single-dose dry powder inhaler (DPI) Breezhaler® (*Novartis International AG,* Basilea, Switzerland). The content of the four hard gelatin capsules was discharged for each experiment and the experiments were performed in triplicate. The flow rate was adjusted to 60 L/min using a DFM 2000 flowmeter (*Copley Scientific, Nottingham,* United Kingdom), and the time duration of the test was of 4 s. The DPI device, nozzle, induction port, impactor stages, and filter were weighed before and after the experiment, and parameters (MMAD, FPF, and ED) were quantified by gravimetry.

Aerodynamic diameter, MMAD, was determined by plotting the cumulative mass percentage for each stage in a probability scale with respect to the aerodynamic diameter of the stage in a log scale. The fine particle fraction (FPF) was established as the percentage of sample <5 $\mu$m, whereas the emitted dose (ED) was the percentage of sample exiting the device and reaching the impactor. Aerodynamic diameter was also confirmed using the Aerosizer® computer software after sample aerosolization in a Aerosizer® (TSI Instruments Ltd. UK) connected to Aero-Disperser®, performing direct flight-time measurements. A small amount of sample was placed in a sampler of the aero-disperser and measurements were performed using a 41 mA laser current, a nozzle type of 200 $\mu$m, a size range of 0.1 a 200 $\mu$m, and a duration of 30 seconds.

The parameters (MMAD, FPF and ED) of the purified pollen capsules prepared according to Example 1 are shown in Table 1.

**Table 1.** *In vitro* aerodynamic properties of the purified pollen capsules prepared according to Example 1. FPF: fine particle fraction; ED: emitted dose; MMAD: mass median aerodynamic diameter; ETR: extrathoracic region; TBR: tracheobronchial region; and PR: pulmonary region. The data represents average values $\pm$ standard deviation, n=3 independent measurements.

| Pollen particle | FPF (%) | ED (%) | MMAD ($\mu$m) | ETR (%) | TBR (%) | PR (%) | Density (g/cm$^3$) |
|---|---|---|---|---|---|---|---|
| Hollow *M. chamomilla* | 10 $\pm$ 3 | 86 $\pm$ 9 | 8 $\pm$ 1 | 84 $\pm$ 3 | 12 $\pm$ 1 | 2 $\pm$ 1 | 0.14 |
| Defatted *M. chamomilla* | 3 $\pm$ 1 | 94 $\pm$ 7 | 16 $\pm$ 2 | 95 $\pm$ 3 | 3 $\pm$ 2 | - | 0.44 |
| Hollow *H. annuus* | 7 $\pm$ 5 | 89 $\pm$ 11 | 11 $\pm$ 4 | 91 $\pm$ 4 | 5 $\pm$ 1 | 2 $\pm$ 1 | 0.13 |
| Defatted *H. annuus* | 2 $\pm$ 1 | 70 $\pm$ 19 | 15 $\pm$ 1 | 78 $\pm$ 4 | 1 $\pm$ 0 | - | 0.38 |

All the capsules showed an optimal emitted dose of the device (between 70-94%). However, these parameters were particularly notable in the case of hollow pollen capsules, where an aerodynamic diameter (MMAD) of 8 and 11 $\mu$m and a FPF of 10 and 7%, respectively, were obtained.

These particle size distributions were further confirmed by means of time-of-flight measurements using an Aerosizer LD. The samples showed similar aerodynamic size distributions for the all the experimental conditions, being 8 $\pm$2 $\mu$m and 14$\pm$1 $\mu$m for hollow and defatted *M. chamomilla* capsules, respectively, and 10$\pm$1 $\mu$m and 16$\pm$1 $\mu$m for hollow and defatted *H. annuus* capsules.

Pollen grains and fungal spores are deposited by inertial impact in the oropharyngeal region and, as shown in Figure 1, most capsules were retained in the extrathoracic region (region A). This was particularly notable in the case of the defatted pollen capsules, where higher densities of the capsules (0.38 g/cm$^3$ for *H. annuus* and 0.44 g/cm$^3$ in the case of *M. chamomilla*) led to greater retention in the inhaler (region 1) and the trachea (region 3), whereas in the case of hollow pollen particles (with densities of 0.13 g/cm$^3$ for *H. annuus* and 0.14 g/cm$^3$ for the case of *M. chamomilla*) could be detected in the pulmonary region (region C).

However, particle distribution in the lungs was inadequate.

Example 6. Aerodynamic study of coated purified pollen capsules

*In vitro* distribution profile of coated capsules prepared as described in Example 2 was evaluated using the Andersen Cascade Impactor as described in Example 5. Parameters (MMAD, FPF and ED) were quantified by gravimetry.

Figure 2 and Figure 3 show the percentage of capsules deposited in the inhaler, mouth, and trachea in stages -1 to 6 and in the filter. Although capsule coating did not significantly influenced the improved exit thereof from the inhaler, a lower proportion of sample was observed both in the trachea and in the first stage of the impactor, compared to uncoated capsules.

The *in vitro* aerodynamic profile calculated by the Inhalytix™ software (Copley Scientific LTD., Nottingham, United Kingdom) is shown in Table 2. This distribution is highly conditioned by aerodynamic diameter decrease. The values of the fine particle fraction were 2.5 times higher after coating. Coated capsules left the first stages and were uniformly distributed in a higher percentage, up to 23%, in the tracheobronchial region. These coated capsules finally reached the last stages of the impactor, which correspond to the pulmonary region, with values of up to 8% for mannitol-coated/leucine-coated *M. chamomilla* capsules, which was 4 times more than uncoated capsules.

**Table 2.** *In vitro* aerodynamic properties of capsules coated with different carriers (Example 2). FPF: fine particle fraction; ED: emitted dose; MMAD: mass median aerodynamic diameter; ETR: extrathoracic region; TBR: tracheobronchial region; and PR: pulmonary region. The data represents average values $\pm$ standard deviation, n=3 independent measurements.

| Carrier | | FPF (%) | ED (%) | MMAD ($\mu$m) | ETR (%) | TBR (%) | PR (%) |
|---|---|---|---|---|---|---|---|
| Leucine | *M. chamomilla* | 26 $\pm$ 10 | 90 $\pm$ 6 | 7 $\pm$ 2 | 72 $\pm$ 4 | 23 $\pm$ 2 | 7 $\pm$ 1 |
| | *H. annuus* | 19 $\pm$ 4 | 88 $\pm$ 16 | 8 $\pm$ 0 | 76 $\pm$ 4 | 17 $\pm$ 2 | 4 $\pm$ 0 |
| Mannitol | *M. chamomilla* | 24 $\pm$ 7 | 89 $\pm$ 5 | 7 $\pm$ 2 | 69 $\pm$ 4 | 21 $\pm$ 2 | 5 $\pm$ 1 |
| | *H. annuus* | 25 $\pm$ 2 | 90 $\pm$ 7 | 8 $\pm$ 0 | 73 $\pm$ 5 | 19 $\pm$ 1 | 6 $\pm$ 0 |
| Leucine/Mannitol | *M. chamomilla* | 27 $\pm$ 14 | 94 $\pm$ 12 | 7 $\pm$ 3 | 72 $\pm$ 5 | 21 $\pm$ 3 | 8 $\pm$ 1 |
| | *H. annuus* | 20 $\pm$ 3 | 85 $\pm$ 9 | 8 $\pm$ 0 | 72 $\pm$ 6 | 17 $\pm$ 2 | 4 $\pm$ 1 |

Example 7. Additional characterization of coated purified pollen capsules

The pollen capsules coated according to the three methods described in Example 2 were characterized by FT-IR. Functional group peaks of hollow *M. chamomilla* capsules could be divided into three regions: (1) A broad signal of 3300 $cm^{-1}$ (O-H vibration), followed by an asymmetrical and symmetrical $CH_2$ and $CH_3$ signal at 2928 and 2854 $cm^{-1}$, respectively; (2) A region between 1800-1500 $cm^{-1}$, where peaks around 1686 and 1579 $cm^{-1}$ correspond to aromatic ketones and the carboxylate group (-COO-), respectively; and (3) The fingerprint region (at 1500-800 $cm^{-1}$), characteristic for each type of pollen. Since sporopollenins constitute a group of phenylpropanoid acid-based biopolymers, they show vibrational spectrum bands due to aromatic ring vibrations at 1515 $cm^{-1}$ (C=C), a signal at about 1056 $cm^{-1}$ (C-C, C-H) which is attributed to carotenoids, and peaks related to C-H bonds in aromatic ring structures at 845, 1170, and 1440 $cm^{-1}$.

The presence of leucine and mannitol pharmaceutically acceptable carriers adhered to the outer surface (sporopollenin) of the capsules could be observed following the method described in Example 2 through the presence of the amino group characteristic of L-leucine, (C-N, 1020-1250 $cm^{-1}$ and $R\text{-}NH_2$ at 1590-1627 $cm^{-1}$) and at 929, 959, 1194, and/or 1209 $cm^{-1}$ for mannitol.

It was observed that the peaks of the capsules did not show significant changes after dry mechanical coating (1). The inventors consider that a possible reason could be the absence of covalent chemical interaction between the pollen capsule and the carrier, but rather an adhesion interaction.

Furthermore, the coated pollen capsules prepared according to Example 2 were also characterized by means of differential scanning calorimetry (DSC) to evaluate the association between the capsules and the carrier. Hollow *M. chamomilla* capsules did not experience any phase change. The DSC curve of leucine, mannitol, and their combination, showed a slight reduction in the height and width of the characteristic endothermic peak, with a reduction in its phase change melting enthalpies (AHm) (Table 3), which suggests a reduction in carrier crystallinity and confirms successful carrier coating in *M. chamomilla* capsules. The amount of associated carrier was also estimated assuming that *M. chamomilla* capsules do not prevent heat release by the carriers. It was observed that the melting enthalpy of the carriers, for dry mechanical coating (solid mixture), reduced significantly compared to lyophilized and spraydried carriers (solid mixture<lyophilization<spray drying). This reduction in latent heat could be attributed to reduced carrier coating which was estimated at 29, 48, and 100% for mannitol; 0, 54, and 86% for leucine, and 42, 25, and 58% for their combination, respectively.

**Table 3.** Temperature (°C) and phase change (J/g) of mannitol (MAN), leucine (LEU), and combination, after the association thereof with hollow *M. chamomilla* capsules, following the methods of Example 2.

| | Pure | | Solid mixture | | Spray drying | | Lyophilization | |
|---|---|---|---|---|---|---|---|---|
| | J/g | T (°C) | J/g | T (°C) | J/g | T (°C) | J/g | T (°C) |
| MAN | -262.19 | 168.83 | -74.47 | 168.00 | -262.31 | 167.66 | -125.56 | 169.33 |
| LEU | -875.09 | 296.66 | - | | -749.07 | 282.66 | -473.59 | 252.33 |
| MAN/LEU | - | | -72.27 | 168.00 | -60.57 | 165.50 | -39.56 | 165.83 |
| | | | -126.30 | 221.83 | -164.76 | 247.66 | -83.10 | 211.33 |

The coating yield was high, with values of almost 100% for dry mechanical coating (solid mixture) and > 90% when performing carrier adhesion in the case of coating by lyophilization. However, spray drying gave a yield of less than 5%.

According to the data from FTIR, DSC, and yield parameters, lyophilization technique was selected for coating hollow *M. chamomilla* capsules.

The effect that the carriers have on the surface of the capsules was likewise evaluated by means of scanning electron microscopy (SEM, Figure 4). The SEM images showed non-aggregated capsules that maintain their spherical geometry and integrity with a corrugated surface, as well as their nanoporous surface.

Example 8. Aerodynamic study of the microparticles for pulmonary release according to the invention

The *in vitro* distribution profile of microparticles for pulmonary release comprising *M. chamomilla* capsules prepared as described in Example 4 was evaluated using the Andersen Cascade Impactor as described in Example 5. Parameters (MMAD, FPF and ED) were quantified by gravimetry. The profile for comparative microparticles was also evaluated: capsules not comprising coating with a pharmaceutically acceptable excipient.

Figure 5 shows the percentage of microparticles for pulmonary release according to the invention (comprising a pollen capsule, a rifabutin nanosystem, and a mannitol/leucine coating) and of comparative uncoated microparticles deposited in the inhaler, mouth, and trachea in stages -1 to 6 and in the filter.

The microparticles which encapsulate the drug showed an increase in their aerodynamic diameter (Table 4). It was observed that coating with carriers significantly reduced the aerodynamic diameter, which reached a size of 8 $\mu$m. The proportion of capsules reaching the tracheal region was between 2 and 3 times higher after using a carrier, reaching values of 20%, compared to <10% in the pulmonary region. These results suggest a greater pulmonary exposure of large porous particles, where the density of the particles, rather than their physical dimension, determines the deposition. These properties allow a more efficient deposition.

**Table 4.** *In vitro* aerodynamic properties of microparticles comprising *M. chamomilla* capsules and protamine nanosystems with 5% (w/w) rifabutin. FPF: fine particle fraction; ED: emitted dose; MMAD: mass median aerodynamic diameter; ETR: extrathoracic region; TBR: tracheobronchial region; and PR: pulmonary region. The data represents average values $\pm$ standard deviation, n=3 independent measurements.

| Carrier | FPF (%) | ED (%) | MMAD ($\mu$m) | ETR (%) | TBR (%) | PR (%) |
|---|---|---|---|---|---|---|
| - | 5 $\pm$ 2 | 74 $\pm$ 10 | 13 $\pm$ 4 | 86 $\pm$ 4 | 6 $\pm$ 1 | - |
| Leucine/Mannitol | 25 $\pm$ 2 | 100 $\pm$ 7 | 8 $\pm$ 0 | 75 $\pm$ 2 | 21 $\pm$ 2 | 8 $\pm$ 1 |

Example 9. Additional characterization of the pollen capsules comprising nanosystems described in Example 4

The nanosystems prepared by means of the solvent displacement technique had a hydrodynamic diameter of 198 $\pm$ 19 nm, a positive surface charge (11 $\pm$ 10 mV), and showed monodispersion (PDI<0.2). Satisfactory encapsulation was obtained with rifabutin incorporation of 42 $\pm$ 7%.

The encapsulation efficiency (EE%), loading capacity (LC%), and load content (w/w) of the samples were calculated using the following equations:

$$\text{Encapsulation efficiency (EE\%)} = \frac{\text{associated nanosystems}}{\text{total nanosystems}} \times 100$$

$$\text{Loading capacity (LC\%)} = \frac{\text{Mass of nanosystems}}{\text{Mass of capsules comprising nanosystems}} \times 100$$

$$\text{Load content (w/w)} = \frac{\text{Mass of nanosystems}}{\text{Mass of capsules}}$$

The encapsulation efficiency in pollen particles (hollow, ratio 1:1) was studied by means of gravimetry and determined to be 66%, whereas the loading capacity of nanocapsules was 71% with respect to the total mass used. In comparison, the encapsulation efficiency and loading capacity of the particles in the case of rifabutin-free nanosystems was 59 and 58, respectively.

By means of confocal microscopy, it was observed that the nanosystems encapsulated in of M. *chamomilla* capsules (prepared according to Example 4) tended to accumulate on the inside, although their internal distribution was not uniform. The use of a higher pressure (50 mbar) allowed obtaining a high association while at the same time ensuring the passage of air through the capsule, which is of interest for the specific application thereof through the pulmonary route.

Figure 6 shows the FTIR (A) and XRPD (B) spectra. FTIR analysis did not reveal any structural alteration of sporopollenin capsules after loading the nanosystems therein, with a distribution in the inner cavity of the pollen through the openings and nanochannels present on the wall of the pollen. The incorporation of rifabutin in the nanosystems facilitated their loading into sporopollenin capsules, compared to the non-encapsulated drug, where the presence of rifabutin was detected at 1068 (secondary alcohol), 1247 (C=O), 1422 and 1372 (C-H), 1602 (C=C) 1647 (N-H), 1668, 1730 (C=O) and 3207 cm$^{-1}$ (N-H).

Nanosystem encapsulation in pollen capsules was also confirmed by means of XRPD analysis. The presence in the XRPD pattern of a broad peak with a low intensity in the 2$\theta$ range indicated the amorphous nature of the pollen particles, whereas rifabutin showed a crystalline structure with peaks at 10.75, 12.63, 15.50, 17.9, 18.9, and 22.8 2-theta. The disappearance of drug peaks confirmed the effective inclusion of the drug in the systems.

Rifabutin nanosystem-loaded pollen capsules were then lyophilized in the presence of mannitol and leucine and characterized by DSC. As can be seen in the thermograms of Figure 7, the mannitol-coated/leucine-coated pollen capsules showed a peak at 155°C corresponding to mannitol, followed by exothermic recrystallization and melting at 165°C, which may correspond to form change from mannitol β to a polymorph δ. The estimated complete association of mannitol and leucine was 35%.

Toxicity

To ensure that the microparticles of the invention can be safely used for pharmaceutical uses, MTT was used to investigate their cytotoxicity in Raw264.7 cells. In that sense, the *in vitro* toxicity of the microparticles of the invention, prepared according to Example 4, was evaluated by means of an MTT colorimetric assay, quantifying the water-insoluble formazan in RAW 264.7 cells after 24 hours of incubation of different particle concentrations. Cells were seeded (10,000 cells/well) 24 h before the experiment in a 96-well plate in a DMEM culture medium (10% FBS and 1% penicillin/streptomycin) at a cell density of 37°C and 5% $CO_2$. The medium was then replaced with the formulations and the cells were incubated for 24 hours. Fresh medium was used as negative control and Triton x100 (1% v/v) was used as positive control. After 24 h, 0.5 mg/mL of MTT dissolved in PBS were added, and the cells were incubated for 4 h at 37°C in darkness. Formazan crystals were dissolved by means of adding HCl 0.04 N in isopropanol and absorbance was measured at 570 nm using a microplate reader (*Synergy H1 Hybrid Multi-Mode, BioTek, Winooski,* VT, USA). Cell viability (%) was calculated following the equation:

$$\text{Cell viability (\%)} = \frac{\text{Sample absorbance} - \text{Negative control absorbance}}{\text{Positive control absorbance} - \text{Negative control absorbance}} \times 100$$

Metabolic activity was quantified by means of formazan determination after incubating the microparticles of the invention and was compared with the activity of non-encapsulated nanosystems. Figure 8 showed a concentration-dependent toxicity, similar in both high-concentration formulations (1 mg/mL, equivalent to 50 μg of rifabutin), but lower for the non-encapsulated nanosystems at lower concentrations. Viability values of 69 + 18% were obtained for the capsules of the invention at concentrations of 0.1 mg/mL. Likewise, a reduction in drug toxicity at high concentrations (rifabutin >50% of viability at 12.5 μg/mL for Raw 264.7) was observed after encapsulation both in the nanosystems and in the pollen platforms.

Dissolution in a pulmonary medium

*In vitro* release of the rifabutin included in the nanosystems from the microparticles of the invention was determined in a simulated lung medium (SLM) composed of NaCl 116 mM, $NaHCO_3$ 27 mM, $C_2H_5NO_2$ 5 mM, $NaH_2PO_4$ ($H_2O$) 1.1 mM, $CaCl_2$ 0.18 mM, and $H_2SO_4$ 0.5 mM in distilled water at pH 7.4. 10 mL of the dissolution medium containing 4 mg of microparticles of the invention (18.4 μg of rifabutin) were kept under stirring at 200 rpm and 37°C. 1 mL aliquots were extracted at preestablished time intervals (5, 10, 15, 20, 30, 40, 50, and 60 min), centrifuged for 1 min at 7500 g, filtered (0.22-micron filters), and replaced with a an equal volume of fresh simulated lung medium. Samples were analyzed by means of spectrophotometer ($\lambda_{max}$ = 237 nm).

*In vitro* dissolution of the rifabutin from the microparticles of the invention (*M. chamomilla*) was evaluated. As shown in Figure 9, the inclusion of nanocapsules in microparticles improved rifabutin dissolution in the medium compared to free rifabutin for the first 40 min, with a total drug release of 29 $\pm$ 7% after 1 h, whereas it was slower compared to rifabutin release from nanosystems not incorporated in microparticles. This can be due to a slower general process involving the initial release of the nanosystems from the pollen platforms and the subsequent dissolution of the drug of the nanosystems in the medium. Statistical difference was not observed between the dissolution of rifabutin of the nanosystems compared to the capsules for up to 50 minutes.

Results were evaluated using mathematical models for drug release kinetics. Todos the experiments were performed in triplicate. *In vitro* drug release kinetics was evaluated using zero order, first order, Higuchi model, and Korsmeyer-Peppas model to elucidate the physical mechanism of drug release.

Drug release pattern was evaluated by means of different kinetic models (Table 5) using the DDSolver software, and the coefficient of determination ($R^2$), adjusted $R^2$, and Akaike information criterion (AIC) were evaluated to select the best-fitting model. It was observed that rifabutin released from nanosystem-loaded capsules best fit the Korsmeyer-Peppas model, which explains drug diffusion from a polymer matrix. The n value of 0.34 suggests Fickian diffusion (n < 0.43).

**Table 5.** Kinetics of the microparticles according to the invention (protamine nanosystems with 5% (w/w) of rifabutin in *M. chamomilla* capsules according to Example 4).

| Parameter | Zero order | First order | Higuchi | Korsmeyer-Peppas |
|---|---|---|---|---|
| $R^2$ | -3.1849 | -2.3413 | 0.0966 | 0.9908 |

(continued)

| Parameter | Zero order | First order | Higuchi | Korsmeyer-Peppas |
|---|---|---|---|---|
| Adjusted $R^2$ | -3.1849 | -2.3413 | 0.0966 | 0.9892 |
| AIC | 49.0335 | 47.1408 | 29.0550 | 7.2045 |
| $K_0$ | 0.6125 | 0.0071 | 4.1352 | 9.3346 |

Example 10. Cellular interaction in the dynamic culture of pollen capsules comprising nanosystems

The shape and function of pulmonary epithelial cells are largely activated by the shear force resulting from the flow resulting from physiological respiration.

In this context, macrophage cells RAW 264.7 and pulmonary epithelial cells of line A549 were cultured in a $\mu$-Slide I Luer fluid chamber to simulate cell interaction in flow conditions, a situation similar to that in the respiratory flow (0.5 dyn/cm³). The cells were cultured under flow for 24 h, supplying them with: i) purified pollen capsules (Example 2); ii) DiD-labelled microparticles according to the invention (capsules + chitosan nanosystem, prepared in a manner similar to that described in Example 3 and Example 4, but substituting protamine for chitosan); and iii) the same nanosystem as in (ii), but without encapsulation in purified pollen capsules (chitosan nanosystem, prepared in a manner similar to that described in Example 3, but substituting protamine for chitosan). Results were studied by flow cytometry and fluorescence microscope.

Purified pollen capsules could interact with cells, being retained by both cell lines even after 24 h of flow (Figure 10). In contrast, when the same experiment was performed with the nanosystem in solution, they were not retained but removed with the perfusion medium, so visible fluorescence was not observed (Figure 11). Moreover, the microparticles according to the invention showed a behavior similar to that of hollow purified pollen capsules, and were retained by the cells (Figure 12). This prolonged interaction allowed the uptake of the nanosystems by the cells (arrows in Figure 10), with red fluorescence being observed inside the cells around the nanosystem-loaded capsules. These results are consistent with the observations made on static culture, which indicates that the microparticles according to the invention acted as a multi-stage release device, by first increasing interaction with cells, followed by subsequent release of the nanosystems.

Example 11. Antibacterial activity of the microparticles of the invention

Efficacy studies - Growth inhibition

The antibacterial activity of the microparticles of the invention with rifabutin (and of the nanosystems) was evaluated in the strain that grows rapidly, *M. phlei,* and in the strain that grows slowly, *M. smegmatis,* using a resazurin-based microdilution assay according to the EUCAST guidelines and CSLI guidelines with slight modifications. This is a quick and objective method, and the minimum inhibitory concentration (MIC) is easily detectable because resazurin (blue) turns into resorufin (pink fluorescence) in the presence of viable bacteria. The microparticles of the invention with rifabutin were diluted in a cation-adjusted 2X Mueller-Hinton broth (CaMHB) until reaching a final antibiotic concentration of 32 mg/L. Since the range of susceptibility of *Mycobacterium* species to rifabutin is ≤0.25-16 mg/L, the highest tested antibiotic concentration was 16 mg/L. A bacterial suspension (in CaMHB) was diluted to a final concentration of $5 \times 10^5$ CFU/mL. For efficacy determination, the following controls were used: (i) growth control (without treatment), (ii) sterility control (CaMHB), (iii) positive control for inhibitory effect (free rifabutin in solution), and as well as (iv) the microparticles of the invention (without rifabutin), so as to take into account the possible effects of the microparticles of the invention on bacterial growth. After an incubation period of 24 h at 37°C, 20 $\mu$L of each well were transferred in triplicate to new 96-well plates containing 100 $\mu$L of 2X CaMHB, 60 $\mu$l of 3X PBS, and 20 $\mu$L of resazurin. Fluorescence intensity was measured at 560 and 590 nm of excitation and monitored using the FLUOR®star Omega multimode lector (Biotron Healthcare, India) and the MARS data analysis software, after an incubation period of 30 min at 37°C. The lowest concentration at which color change was observed was considered the minimum inhibitory concentration (MIC) value. Furthermore, IC50 was calculated using an online tool: "Quest Graph™ IC50 Calculator". AAT Bioquest, Inc.

Biofilm disruption studies

In these experiments, the antimicrobial potential of the system was evaluated in *Mycobacterium smegmatis* used as a model of *M. tuberculosis* as they have homologous genes. The biofilm formation potential of *Mycobacterium smegmatis* was evaluated by scanning electron microscopy and the formation thereof was quantified with crystal violet, using 96-well plates made of polyvinyl chloride.

To view the bacteria, as well as the possible biofilm, the bacteria were cultured on polylysine-treated glass, and primary fixing with 2.5% glutaraldehyde in phosphate buffer (0.2 M; 1 hour, 4°C) was performed after the experiment.

Bacterial cell dehydration was then performed using increasing ethanol concentrations. The FESEM Ultra Plus microscope (Zeiss, Germany) was used to view the bacteria.

A crystal violet-based spectrophotometric study was used. To that end, *M. smegmatis* was inoculated in liquid culture (5 mL of 7H9 medium supplemented with OACC) and left to grow to exponential phase. The culture was diluted 1:100 and 100 $\mu$L were pipetted into each well. The controls were the rifabutin-free formulations and the blank was the bacterial culture-free medium. After incubating for two days at 37°C and once the biofilm was formed, the plates were washed with distilled water to remove planktonic cells and 200 $\mu$L of each formulation were added in serial dilutions (in the same manner as above). After 24 h, the plate was washed with water again to remove possible planktonic cells, and the biofilm was stained with 125 $\mu$L of 0.1% crystal violet solution and incubated for 10 min at room temperature. It was the washed with water and stirred vigorously. Finally, remaining crystal violet was solubilized by adding 200 $\mu$L of 95% ethanol and incubating for 15 min at room temperature. The solution was then transferred to a 96-well, flat-bottom plate, and biofilm formation was measured at 570 nm.

## Antimicrobial activity studies in *M. phlei and M. smegmatis*

Studies on the antibacterial potential of the microparticles of the invention were performed in *M. phlei and M. smegmatis.* In both cases, the microparticles of the invention, and the nanosystems, without drug did not have any inhibitory potential. When comparing the different study times, it can be verified that the formulations always have a higher antibacterial potential than the rifabutin-free drug and that furthermore this potential improves even further after the association of the formulations with purified pollen capsules (Figure 13, Table 6, and Table 7).

Table 6. Minimum inhibitory concentration (MIC), IC50, and IC90 of chitosan and protamine nanosystems.

| | Preinoculum culture t=0 | | | 2-day prelnoculum | | | 4-day preinoculum | | | Biofilm culture | | |
|---|---|---|---|---|---|---|---|---|---|---|---|---|
| | MIC | IC50 | IC90 | MIC | IC50 | IC90 | MIC | IC50 | IC90 | MIC | IC50 | IC90 |
| NC-CS-PEG | 0.5 | 0.21 | 1.89 | 0.5 | 0.27 | 2.43 | 0.5 | 0.33 | 2.97 | 0.5 | 0.34 | 3.06 |
| NC-CS-LEC | 0.5 | 0.12 | 1.08 | 0.5 | 0.24 | 2.16 | 0.5 | 0.25 | 2.25 | 0.5 | 0.19 | 1.71 |
| NC-Prota | 0.5 | 0.21 | 1.89 | 0.5 | 0.21 | 1.89 | 0.5 | 0.24 | 2.16 | 0.5 | 0.13 | 1.17 |
| Rifabutin | 1 | 0.34 | 3.06 | 4 | 2.24 | 20.16 | 4 | 2.04 | 18.36 | 4 | 4.67 | 42.03 |

Table 7. Minimum inhibitory concentration (MIC), IC50, and IC90 of the microparticles of the invention (chitosan or protamine nanosystems in purified pollen capsules).

| | Preinoculum culture t=0 | | | 2-day preinoculum | | | 4-day prelnoculum | | | Biofilm culture | | |
|---|---|---|---|---|---|---|---|---|---|---|---|---|
| | MIC | IC50 | IC90 | MIC | IC50 | IC90 | MIC | IC50 | IC90 | MIC | IC50 | IC90 |
| NC-CS-PEG | 0.5 | 0.21 | 1.89 | 0.5 | 0.27 | 2.43 | 0.5 | 0.33 | 2.97 | 0.5 | 0.34 | 3.06 |
| NC-CS-LEC | 0.5 | 0.12 | 1.08 | 0.5 | 0.24 | 2.16 | 0.5 | 0.25 | 2.25 | 0.5 | 0.19 | 1.71 |
| NC-Prota | 0.5 | 0.21 | 1.89 | 0.5 | 0.21 | 1.89 | 0.5 | 0.24 | 2.16 | 0.5 | 0.13 | 1.17 |
| Rifabutin | 1 | 0.34 | 3.06 | 4 | 2.24 | 20.16 | 4 | 2.04 | 18.36 | 4 | 4.67 | 42.03 |

| | Plate preinoculum culture | | | 2-day preinoculum | | | 4-day preinoculum | | | Biofilm culture | | |
|---|---|---|---|---|---|---|---|---|---|---|---|---|
| | MIC | IC50 | IC90 | MIC | IC50 | IC90 | MIC | ICSO | IC90 | MIC | IC50 | IC90 |
| Pollen+ NC-CS-PEG | 0.5 | 0.11 | 0.99 | 0.5 | 0.24 | 2.16 | 0.5 | 0.31 | 2.79 | 0.5 | 0.16 | 1.44 |

(continued)

| | Plate preinoculum culture | | | 2-day preinoculum | | | 4-day preinoculum | | | Biofilm culture | | |
|---|---|---|---|---|---|---|---|---|---|---|---|---|
| | MIC | IC50 | IC90 | MIC | IC50 | IC90 | MIC | ICSO | IC90 | MIC | IC50 | IC90 |
| Pollen + NC-CS-LEC | 0.25 | 0.08 | 0.72 | 0.5 | 0.21 | 1.89 | 0.5 | 0.16 | 1.44 | 1 | 0.08 | 0.72 |
| Pollen+ NC-Prota | 1 | 0.12 | 1.08 | 0.5 | 0.24 | 2.16 | 0.25 | 0.08 | 0.72 | 1 | 0.10 | 0.90 |
| Pollen + Rifabutin | 1 | 0.59 | 5.31 | 4 | 2.19 | 19.71 | 4 | 1.63 | 14.67 | 4 | 4.57 | 41.13 |

Biofilm inhibition and disruption in *M. smegmatis*

Crystal violet was used to know which was the biofilm dispersion capacity of the system. Results (Figure 14 and Tables 6 and 7) indicate that rifabutin in suspension (not encapsulated) requires a higher concentration than encapsulated rifabutin to achieve bacterial biofilm disruption. It is again concluded that efficacy improves even further after the association thereof with purified pollen capsules and that this effect is more pronounced at longer growth times (i.e., IC90 values 40 times lower with respect to the non-encapsulated antibiotic).

Morphological analysis of the bacterial biofilm

The amount 2 mg/mL was selected to view the effect, since there were hardly any differences between the culture with rifabutin in suspension or without treatment at that drug concentration; however, the formulations (nanosystems and purified pollen capsules with nanosystems) indeed achieve a biofilm disruption effect of more than 80%.
Therefore, as can be seen in Figure 15, the bacterial cells showed wall and size deformations, with some of them appearing completely empty. It can be seen that when studying the effect of drug-free formulations, these formulations did not cause any effect on the bacteria under study.

**Claims**

1.   A microparticle for pulmonary release comprising:

- a purified pollen capsule, which comprises an intine layer and an exine layer; and
- at least one nanosystem encapsulated in said capsule, wherein the nanosystem comprises a pharmaceutically active compound;

wherein the exine layer is coated with a pharmaceutically acceptable excipient.

2.   The microparticle according to claim 1, **characterized in that** the pharmaceutically acceptable excipient constitutes the outermost layer of the microparticle.

3.   The microparticle according to any of claims 1 or 2, **characterized in that** the pharmaceutically acceptable excipient reduces the mass median aerodynamic diameter of the microparticle by at least 10%, when compared to an identical microparticle but without a pharmaceutically acceptable excipient, using an Andersen Cascade Impactor.

4.   The microparticle according to any of claims 1 to 3, **characterized in that** the pharmaceutically acceptable excipient is selected from the group consisting of amino acids, peptides, proteins, polymers polyols, carbohydrates, fatty acids, fatty acid salts, phospholipids, and combinations thereof.

5.   The microparticle according to any of claims 1 to 4, **characterized in that** the pharmaceutically acceptable excipient is selected from the group consisting of mannitol, leucine, trehalose, lactose, albumin, dipalmitoylphosphatidylcholine, magnesium stearate, and combinations thereof.

6. The microparticle according to any of claims 1 to 5, **characterized in that** the pharmaceutically acceptable excipient is selected from the group consisting of mannitol, leucine, and combinations thereof.

7. The microparticle according to any of claims 1 to 6, **characterized in that** the pharmaceutically acceptable excipient is in an amount comprised between 10% and 95% by weight, with respect to the weight of the microparticle of the invention without the coating.

8. The microparticle according to any of claims 1 to 7, **characterized in that** the nanosystem comprises polypeptides, polysaccharides, and/or polyamino acids.

9. The microparticle according to any of claims 1 to 8, **characterized in that** the nanosystem comprises chitosan or protamine.

10. The microparticle according to any of claims 1 to 9, **characterized in that** the nanosystem comprises a protamine layer encapsulating the pharmaceutically active compound.

11. The microparticle according to any of claims 1 to 10, **characterized in that** the nanosystem presents a hydrodynamic diameter comprised between 100 and 500 nm, measured by means of dynamic light scattering.

12. The microparticle according to any of claims 1 to 11, **characterized in that** the nanosystem comprises an oily component.

13. The microparticle according to any of claims 1 to 12, **characterized in that** the pharmaceutically active compound is an antibiotic.

14. The microparticle according to any of claims 1 to 13, **characterized in that** the pharmaceutically active compound is an antibiotic for treating tuberculosis.

15. The microparticle according to any of claims 1 to 14, **characterized in that** the pharmaceutically active compound is in a concentration comprised between 0.5% and 20% by weight, with respect to the weight of the nanosystem.

16. The microparticle according to any of claims 1 to 15, **characterized in that** the pharmaceutically active compound is selected from the group consisting of insulin and rifabutin.

17. The microparticle according to any of claims 1 to 16, **characterized in that** the purified pollen capsule is devoid of its lipid layer.

18. The microparticle according to any of claims 1 to 17, **characterized by** a mass median aerodynamic diameter of 1 to 10 $\mu$m.

19. A method for the preparation of a microparticle as defined in any of the preceding claims, **characterized in that** it comprises:

(a) a washing stage which comprises washing a pollen particle;
(b) a stage which comprises treating the pollen particle obtained in the preceding stage with an acidic medium to obtain hollow purified pollen capsules;
(c) a stage which comprises incubating the hollow capsules obtained in the preceding stage with a nanosystem comprising a pharmaceutically active compound; and
(d) a stage which comprises coating the resulting capsules with a pharmaceutically acceptable excipient.

20. A microparticle for pulmonary release, **characterized in that** it is obtained according to the method of claim 19.

21. A pharmaceutical composition, **characterized in that** it comprises a microparticle according to any of claims 1 to 18, or an obtainable microparticle which can be obtained according to claim 20, and a pharmaceutically acceptable excipient.

22. The microparticle according to any of claims 1 to 18, or an obtainable microparticle which can be obtained according to claim 20, or the pharmaceutical composition according to claim 21, for use as a medicinal product.

23. The microparticle according to any of claims 1 to 18, or an obtainable microparticle which can be obtained according to claim 20, or the pharmaceutical composition according to claim 21, for use in the treatment of lung-related diseases.

24. The microparticle, obtainable microparticle, or pharmaceutical composition, for use according to claim 23, wherein the lung-related diseases are selected from the group consisting of asbestosis, asthma, bronchiectasis, bronchitis, bronchiolitis, chronic cough, chronic obstructive pulmonary disease (COPD), cystic fibrosis, idiopathic pulmonary fibrosis (IPF), interstitial pulmonary disease (IPD), lung cancer, pleuritis, pneumonia, pulmonary embolism, pulmonary fibrosis, pulmonary hypertension, sarcoidosis and viral or bacterial diseases.

25. The microparticle, obtainable microparticle, or pharmaceutical composition, for use according to any of claims 23 or 24, wherein the disease is tuberculosis.

26. The microparticle, obtainable microparticle, or pharmaceutical composition, for use according to any of claims 23 to 25, wherein the treatment is performed through delivery by means of a nebulizer, a pressurized metered-dose inhaler, or a dry powder inhaler, preferably a dry powder inhaler.

Figure 1

Figure 2

Figure 3

Figure 4

Figure 5

Figure 6

Figure 7

Figure 8

Figure 9

Figure 10

Figure 11

Figure 12

Figure 13

Figure 14

## INTERNATIONAL SEARCH REPORT

| | International application No. |
|---|---|
| | PCT/ES2024/070439 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

INVENES, EPODOC, WPI, EMBASE, BIOSIS, MEDLINE, NPL, XPESP, PATENW

**C. DOCUMENTS CONSIDERED TO BE RELEVANT**

| Category* | Citation of document, with indication, where appropriate, of the relevant passages | Relevant to claim No. |
|---|---|---|
| A | WO 2018146365 A1 (UNIV SANTIAGO COMPOSTELA) 16/08/2018, page 5, line 10 page 6, line 24; page 9, line 26 page 18, line 19; examples. | 1-26 |
| A | ROBLA SANDRA et al.; A ready-to-use dry powder formulation based on protamine nanocarriers for pulmonary drug delivery. European Journal Of Pharmaceutical Sciences : Official Journal Of The European Federation For Pharmaceutical Sciences Netherlands 01 Jun 2023, 01/06/2023, Vol. 185, pages 106442, ISSN 1879-0720 (Electronic), <DOI: 10.1016/j.ejps.2023.106442 pubmed:37019308> | 1-26 |
| A | US 5275819 A (AMER MOH S et al.) 04/01/1994, column 2, line 44-column 6, line 52; column 7, line 40-column 10, line 25; examples. | 1-26 |

☒ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 22/11/2024 | **(25/11/2024)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | N. Vera Gutierrez<br><br>Telephone No. 913495544 |

Form PCT/ISA/210 (second sheet) (July 2022)

## INTERNATIONAL SEARCH REPORT

| International application No. |
| --- |
| PCT/ES2024/070439 |

C (continuation).                    DOCUMENTS CONSIDERED TO BE RELEVANT

| Category * | Citation of documents, with indication, where appropriate, of the relevant passages | Relevant to claim No. |
| --- | --- | --- |
| A | WO 2017010945 A1 (UNIV NANYANG TECH) 19/01/2017, paragraphs [0020]-[0024], [0078], [00145], [00147], [00188]; examples 2, 3. | 1-26 |
| A | US 2009246125 A1 (ATKIN STEPHEN LAWRENCE *et al.*) 01/10/2009, example 4, 8; paragraph [0035]. | 1-26 |
| A | WO 2021183735 A1 (UNIV LELAND STANFORD JUNIOR) 16/09/2021, page 9, line 30-page 10, line 6; page 12, lines 10-20; page 22, lines 7-30; page 27, lines 11-29; examples. | 1-26 |

Form PCT/ISA/210 (continuation of second sheet) (July 2022)

## INTERNATIONAL SEARCH REPORT

| International application No. |
| --- |
| PCT/ES2024/070439 |

CLASSIFICATION OF SUBJECT MATTER

*A61K9/50* (2006.01)
*A61K9/72* (2006.01)
*A61K47/46* (2006.01)
*A61K47/18* (2017.01)
*A61K47/26* (2006.01)
*A61K31/438* (2006.01)
*A61P31/06* (2006.01)

Form PCT/ISA/210 (extra sheet) (July 2022)

## INTERNATIONAL SEARCH REPORT

Information on patent family members

| International application No. |
| --- |
| PCT/ES2024/070439 |

| Patent document cited in the search report | Publication date | Patent family member(s) | Publication date |
| --- | --- | --- | --- |
| US5275819 A | 04.01.1994 | US5013552 A | 07.05.1991 |
| | | WO9219229 A1 | 12.11.1992 |
| | | EP0583238 A1 | 23.02.1994 |
| | | EP0583238 A4 | 08.06.1994 |
| | | CA2102211 A1 | 03.11.1992 |
| WO2017010945 A1 | 19.01.2017 | TW201717889 A | 01.06.2017 |
| US2009246125 A1 | 01.10.2009 | WO2006064227 A1 | 22.06.2006 |
| | | EP1824524 A1 | 29.08.2007 |
| WO2021183735 A1 | 16.09.2021 | NONE | |
| WO2018146365 A1 | 16.08.2018 | US2020129575 A1 | 30.04.2020 |
| | | EP3581174 A1 | 18.12.2019 |
| | | EP3581174 A4 | 30.12.2020 |
| | | ES2613586 A1 | 24.05.2017 |
| | | ES2613586 B2 | 14.05.2018 |
| | | ES2613585 A1 | 24.05.2017 |
| | | ES2613585 B2 | 19.02.2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018146365 A
- WO 2021183735 A
- WO 2005000280 A
- WO 2018146365 A1
- US 5275819 A

### Non-patent literature cited in the description

- **MEHTA, P** ; **BOTHIRAJA, C** ; **KADAM, S** ; **PAWAR, A**. Potential of Dry Powder Inhalers for Tuberculosis Therapy: Facts, Fidelity and Future. *Artif. Cells, Nanomedicine, Biotechnol*, 2018, vol. 46, S791-S806
- **ROBLA, S et al.** A ready-to-use dry powder formulation based on protamine nanocarriers for pulmonary drug release. *European Journal of Pharmaceutical Sciences*, 2023, vol. 185, 106442
- **DIEGO-TABOADA, A** ; **BECKETT, S.T** ; **ATKIN, S.L** ; **MACKENZIE, G**. Hollow Pollen Shells to Enhance Drug Release. *Pharmaceutics*, 2014, vol. 6, 80-96
- *CHEMICAL ABSTRACTS*, 9005-64-5
- *CHEMICAL ABSTRACTS*, 9005-67-8
- *CHEMICAL ABSTRACTS*, 9005-65-6
- *CHEMICAL ABSTRACTS*, 61791-12-6
- *CHEMICAL ABSTRACTS*, 151-21-3
- *CHEMICAL ABSTRACTS*, 9003-11-6
- **AGEITOS, J.M. et al.** Purification of Hollow Sporopollenin Microcapsules from Sunflower and Chamomile Pollen Grains. *Polymers (Basel*, 2021, vol. 13, 2094
- **THWALA, L.N** ; **BELOQUI, A.** ; **CSABA, N.S.** ; **GONZÁLEZ-TOUCEDA, D** ; **TOVAR, S.** ; **DIEGUEZ, C** ; **ALONSO, M.J** ; **PRÉAT, V**. The Interaction of Protamine Nanocapsules with the Intestinal Epithelium: A Mechanistic Approach. *J. Control. Release*, 2016, vol. 243, 109-120
- **ROUCO** ; **ROUCO, H.** ; **DIAZ-RODRIGUEZ, P.** ; **GASPAR, D.P** ; **GONGALVES, L.M.D.** ; **CUERVA, M.** ; **REMUÑÁN-LÓPEZ, C** ; **ALMEIDA, A.J.** ; **LANDIN, M et al.** Rifabutin-Loaded Nanostructured Lipid Carriers as a Tool in Oral Anti-Mycobacterial Treatment of Crohn's Disease. *Nanomaterials*, 2020, vol. 10, 1-18